(19) Europäisches Patentamt
European Patent Office
Office européen des brevets

(11) Publication number: 0 112 117
A2

(12) # EUROPEAN PATENT APPLICATION

(21) Application number: 83307412.3

(51) Int. Cl.³: C 07 C 1/04

(22) Date of filing: 06.12.83

(30) Priority: 14.12.82 US 449586

(43) Date of publication of application: 27.06.84 Bulletin 84/26

(84) Designated Contracting States: BE DE FR GB NL SE

(71) Applicant: KING-WILKINSON PROJECT SERVICES, INC.
5718 Westheimer Suite 200
Houston Texas 77057(US)

(72) Inventor: Hoffman, Edward J.
1805 Bill Nye Street
Laramie Wyoming 82070(US)

(74) Representative: Warden, John Christopher et al,
R.G.C. Jenkins & Co. 12-15, Fetter Lane
London EC4A 1PL(GB)

(54) **Carbonaceous material conversion process.**

(57) Coal or other hydrogen-deficient carbonaceous materials are reacted with steam to produce methane, higher hydrocarbons, and/or oxygenated products. Provision is made for the alkali capture of liberated sulfur compounds, along with partial conversion in a heated pretreatment section. The further processing involves either catalytic conversion in a multiphase reactor-contactor using a methanation or Fischer-Tropsch type catalyst, or additional conversion in holdup and backup sections followed by methanation or Fischer-Tropsch type syntheses. Systems are provided for regenerating the alkali and recovering sulfur, for recovering other compounds, and for activating and regenerating the methanation or Fischer-Trosch type catalyst in place or in an external regenerator. Methods are also provided for recovering, reconstituting, and reactivating catalyst carryover.

EP 0 112 117 A2

FIG. 1

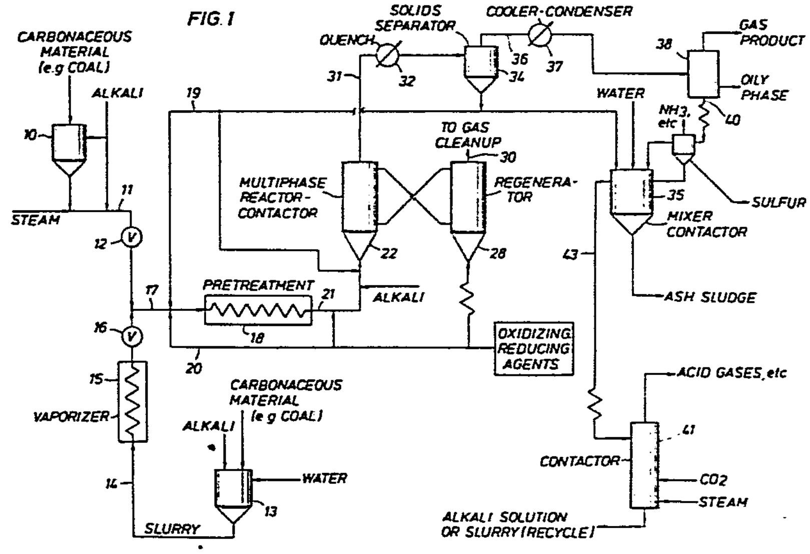

CARBONACEOUS MATERIAL (e.g COAL)
ALKALI
10
11
STEAM
12
V
19
17
16
15
VAPORIZER
14
SLURRY
ALKALI
CARBONACEOUS MATERIAL (e g COAL)
WATER
13
PRETREATMENT
18
21
20
ALKALI
OXIDIZING REDUCING AGENTS
MULTIPHASE REACTOR-CONTACTOR
22
REGENERATOR
28
TO GAS CLEANUP
30
QUENCH
31
32
SOLIDS SEPARATOR
34
36
COOLER-CONDENSER
37
38
GAS PRODUCT
OILY PHASE
40
NH3, etc
WATER
SULFUR
35
MIXER CONTACTOR
ASH SLUDGE
43
ACID GASES, etc
41
CONTACTOR
CO2
STEAM
ALKALI SOLUTION OR SLURRY (RECYCLE)

# CARBONACEOUS MATERIAL CONVERSION PROCESS

## BACKGROUND OF THE INVENTION

The successful thermochemical production of synthetic fuels from hydrogen-deficient carbonaceous materials and steam lies in that combination of reactants, reaction conditions, catalysts and equipment to yield the desired products at optimal efficiency.

Pertinent prior art is U.S. Patent 3,505,204 issued to Edward J. Hoffman.

Integral to this successful operation is the control of the course of conversion at the successive stages, the capture and recovery of sulfur indigenous to the feed materials, and the maintenance of the activity of the catalysts utilized.

By synthetic fuels is meant the range of combustible gaseous, liquid, and solid products achieved as the result of conversions from carbonaceous materials. These synthetic fuel products are simultaneously produced, or cogenerated, with non-fuel products, principally carbon dioxide, but also hydrogen sulfide, nitrogen and/or ammonia, or other derivatives from the components of the feed material.

Included as gaseous synthetic fuel products are hydrogen, carbon monoxide and methane, notably. Liquid products include the increasingly heavier hydrocarbons, and also oxygenated compounds of simple or complex structure. These may arise as the carryover of pyrolysis products and from chemical combinations or re-combinations of other intermediate products. Also known as oils and tars, the liquid product makeup will depend upon the particular process involved.

Finally, there is the solid residue, known variously as char, char-ash or ash-char, or coke. Its combustibility is uncertain, depending upon the degree of conversion. Ideally, only the mineral constituents (the ash) are left and this corresponds to 100% conversion of the organic or carbonaceous makeup of the feed material.

The final mix of all components and fractions will depend upon the process route, the operating conditions and other variables involved in the process.

A naturally-occurring carbonaceous material such as coal consists mainly of carbon, hydrogen and oxygen, with lesser amounts of sulfur and nitrogen, depending upon its origin, plus the ash content. The

hydrogen and oxygen will in part already be bound or combined as water, or will, in part, form water during a conversion process.

For simplicity, a hydrogen-deficient carbonaceous material may be represented simply as C, for carbon. Upgrading involves the introduction of hydrogen (H or $H_2$), directly or indirectly. The hydrogen is most readily derived from steam, denoted as $H_2O(g)$. Alternately, hydrogen itself (as molecular hydrogen, $H_2$) is valuable as a fuel, as is carbon monoxide (CO), as well as methane ($CH_4$) and the other higher hydrocarbons. Oxygenated products such as methanol ($CH_3OH$) also serve as fuels in varying degree.

Which brings up the point that synthetic fuels are what the name implies: substitutes for more conventional fuels. In fact, in cases, the fuel itself is made up of the same components -- on the one hand being obtained "synthetically" via thermochemical conversion methods or otherwise, on the other hand occurring naturally.

In some instances, as in the case of methane or high-Btu gas, full substitution can be made for natural gas provided the Btu-rating is above approximately 900 Btu per cubic foot and other specifications are met (e.g., moisture content, sulfur content

and $CO_2$ content). In other cases, such as burning CO and $H_2$, or $H_2$ alone, the burner must be redesigned to prevent flashback, to assure proper air/fuel ratios, etc. Interestingly, in combusting lower-Btu gases such as CO, or CO in nitrogen (producer gas), the Btu-rating based on the volume of combustion products is not much different than for high-Btu gas. Thus there is a degree of compatibility in combustion systems --though rates and flame temperatures will vary.

Carbon dioxide ($CO_2$) produced as a byproduct or coproduct of the conversion process is of increasing value for enhanced oil recovery. That is, carbon dioxide injected under pressure into earth formations containing heavy crudes, will induce flow of fluids, resulting in additional production.

There is, therefore, a number of uses for the products of syn-fuel conversions. The choices of processing will depend upon the end purposes and the efficiency of the operation. The several processes utilizing steam can be compared as to reaction stoichiometry.

Comparison of Reaction Stoichiometries

Reactions involving steam may be represented symbolically as follows, and which occur to a degree

in most circumstances:

$$C + H_2O(g) \longrightarrow CO + H_2 \qquad .........(1)$$

$$CO + H_2O(g) \longrightarrow CO_2 + H_2 \qquad ........(2)$$

$$C + 2\ H_2O(g) \longrightarrow CO_2 + H_2 \quad \text{(overall)}........(3)$$

The first reaction (1), called steam-gasification or the water-gas reaction, will produce a medium-Btu fuel gas or synthesis gas. This reaction is highly endothermic, requiring heat to be added to sustain the reaction. The heat may be added externally, or internally, for example, by the injection of oxygen to combust a part of the carbonaceous material.

Depending upon temperature and steam concentration, the second reaction (2) -- called the Co-shift, or carbon monoxide-shift, -- will also make an appearance. It is favored by lower temperatures. It is somewhat exothermic and liberates heat.

Also making an appearance will be hydrogen sulfide, produced during the thermal decomposition of the coal or carbonaceous material. Naturally-occurring sulfur in the carbonaceous material structure is liberated in the form of hydrogen sulfide at the partial reducing conditions which exist. At higher temperatures, sulfides or other sulfur compounds in the ash may also be converted to hydrogen sulfide. Its removal is usually required at this

point, to produce a sulfur-free fuel gas or synthesis gas for further processing.

The second reaction (2), the CO-shift, may be conducted in a separate reactor stage to further adjust the proportions of CO and $H_2$, to be used as synthesis gas for further conversion. Hydrogen sulfide removal is generally practiced ahead of the CO-shift to protect the catalyst used in the process. Any carbon dioxide present, also being an acid gas, is also removed as a rule.

The third or overall reaction (3) will go at appropriate conditions with appropriate catalysts to yield $CO_2$ and $H_2$ -- for the appropriate purposes. This may be referred to as the direct production of hydrogen from coal-steam systems.

The further production of hydrocarbons is illustrated by the following sequence:

$$C + H_2O(g) \longrightarrow CO + H_2 \quad \text{....(4)}$$

$$\tfrac{1}{2}\ CO + H_2O(g) \longrightarrow CO_2 + H_2 \quad \text{....(5)}$$

$$\tfrac{1}{2}\ CO + 3/2\ H_2 \xrightarrow{cat.} \tfrac{1}{2}\ CH_4 + \tfrac{1}{2}\ H_2O(g) \quad \text{....(6)}$$

---

$$C + H_2O(g) \longrightarrow \tfrac{1}{2}\ CH_4 + \tfrac{1}{2}\ CO_2 \quad \text{(overall).(7)}$$

The third reaction (6), using the adjusted proportions of $CO/H_2$, produces the methane by employing nickel catalysts. Other proportions and other

catalysts will yield still other such products as higher hydrocarbons. Note that the reaction sequence uses three steps or stages. Furthermore, interstage cooling and heating is required for the different temperature (and pressure) levels necessary. Interstage cooling is also required for hydrogen sulfide removal prior to the CO-shift, and particularly ahead of the third reaction (6), the methanation reaction, in which the catalyst may be poisoned by sulfur. Even with waste-heat recovery, there are marked efficiency losses, and for these and other reasons, simpler conversion routes are in order.

From another standpoint, consider

$$C + H_2O(g) \longrightarrow CO + H_2 \quad \text{.........(8)}$$

$$CO + H_2 \xrightarrow{cat.} \tfrac{1}{2} CH_4 + \tfrac{1}{2} CO_2 \quad \text{.........(9)}$$

$$C + H_2O(g) \longrightarrow \tfrac{1}{2} CH_4 + \tfrac{1}{2} CO_2 \quad \text{(overall).....(10)}$$

The second reaction (9) is sometimes called direct methanation. At these 1/1 $CO/H_2$ proportions, with proper catalysts and conditions, it is possible to avoid the CO-shift conversion step. It is still necessary to cool between stages for sulfur removal, however, unless sulfur-resistant methanation catalysts can be used.

Finally, consider the reaction

$$C + H_2O(g) \xrightarrow{cat.} 1/2\ CH_4 + 1/2\ CO_2 \quad .........(11)$$

This is sometimes called direct catalytic conversion. It occurs in the presence of alkali and nickel catalysts, or other catalysts, at varying pressures. While conversion can occur at lower temperatures, say 700-1200°F, the rate is slow. The conversion is best at temperatures from 1200-1500°F, and preferably 1300-1400°F.

For successful long-term continuous operations, sulfur control is mandatory --as well as control of catalyst activity. These are areas at which this invention is directed.

Also of note is the fact that $CO_2$ and hydrogen will methanate. Thus consider:

$$C + 2\ H_2O(g) \longrightarrow CO_2 + 2\ H_2 \quad ...........(12)$$

$$1/2\ CO_2 + 2\ H_2 \xrightarrow{cat.} 1/2\ CH_4 + H_2O(g) \quad .........(13)$$

---

$$C + H_2O(g) \longrightarrow 1/2\ CH_4 + 1/2\ H_2O(g) \text{ (overall)}..(14)$$

By adjusting $CO_2/H_2$ ratios, and/or by using appropriate catalysts and conditions, it is possible to obtain the above reactions. Similarly, with appropriate catalysts and conditions, higher hydrocarbons and/or oxygenated compounds can be produced.

Application to the specific circumstances with

control of sulfur and catalyst activity is as follows.

Overall Features Of The Invention

The initiation of the reaction between steam and coal or other carbonaceous materials is enhanced by the presence of alkalies. Reactivity is favored by using pulverized coal entrained in steam as the reactants. The alkali is impregnated upon the coal (from solution or suspension), or introduced dry, or introduced separately as a slurry or solution.

By applying heat and assuring a sufficient residence time at temperature in a pretreatment step, thermal degradation proceeds, resulting in the liberation of hydrogen sulfide and other compounds which are captured by the alkali. The use of sodium carbonate or its ores, or sodium hydroxide (or sodium oxide), enhances capture at the temperature levels occurring. A potassium compound is not effective.

The thermal degradation or decomposition is caused by the addition of heat, directly or indirectly, at the same time causing a partial reaction between the steam and carbonaceous material to yield CO and $H_2$. This reaction is endothermic, producing a lowering of the temperature unless heat is further added.

In turn, injection of the coal-steam-alkali mixture into a bed of ebullating nickel catalyst particles, or other appropriate catalysts, will produce conversion to methane and carbon dioxide. With other catalysts and conditions, higher hydrocarbons and/or oxygenated compounds can also be produced -- even hydrogen.

Whereas the conversion

$$C + H_2O(g) \longrightarrow 1/2\ CH_4 + CO2 \quad ............(15)$$

is essentially autothermal or the thermally neutral, the conversion

$$CO + H_2 \longrightarrow 1/2\ CH_4 + 1/2\ CO_2 ............(16)$$

or

$$CO + 3\ H_2 \longrightarrow CH_4 + H_2O(g) \quad ........(17)$$

is highly exothermic -- that is, liberates heat. Thus, control is required between temperature and residence time in the pretreatment section in order to maintain the appropriate reactor temperature. As indicated, the appropriate temperature in the reactor proper is preferably in the range of 1200-1500°F. This will insure sufficient reactivity, at the same time reducing sulfur problems with nickel or other catalysts. That is, sulfide poisoning is reduced at higher temperatures. At the same time, sulfur capture is enhanced in the pretreatment sections by

appropriate residence times and temperatures. It is the optimum combination and tradeoff which ensures successful operation. This will vary with the particular coal or carbonaceous material, and with the catalysts employed.

While the catalyst can be recyled to a regenerator vessel for removal of any sulfur by oxidation or reduction procedures, followed by reactivation if necessary, this all may be carried out internally by the injection or recycle of steam, and/or $CO_2$ as oxidizing agents -- and/or by hydrogen or CO as reducing agents. In short, control of the reactant and product compositions will also maintain catalyst activity.

The alkali carryover is regenerated by dissolution in water followed by injecting steam and carbon dioxide. This reconverts the sulfide to the carbonate for recycle, liberating $H_2S$ for further processing -- namely conversion to elemental sulfur, or combustion to sulfur oxides followed by absorption by limestone or dolomite or their equivalent. The recycled alkali may be evaporated from solution, or injected as a slurry or solution.

As mentioned, sulfided nickel or other catalysts yield to regeneration by oxidation with steam, $CO_2$ or other oxidizing agents -- or by reduction with

$H_2$ or CO, or other reducing agents. This may be accomplished starting with the dry solid form, or by forming alkaline or acidic solutions.

For ablated nickel or other metal catalyst carryover with the ash particulates, the catalyst may be dissolved in acids or alkalies and further treated to yield the solid oxide or other form. Inasmuch as other components may also dissolve, adding to the complexity of treatment, magnetic methods may be employed. For instance, nickel, being paramagnetic, can be recovered intermediately between ferromagnetic materials (e.g., iron) and non-magnetic components. The magnetic susceptibility is enhanced by heat treatment.

Classification, flotation and other extractive metallurgical processes also may suffice.

Alternately, if in appropriate concentrations and quantities, the nickel-containing material may be sent to nickel refining and reprocessing facilities.

The totality of the combined elements as outlined constitutes this invention.

## SCOPE OF INVENTION

The following combination(s) of features constitutes the scope of this invention. It is this

totality which provides the realization of the invention.

Pulverized coal or other carbonaceous materials are entrained or suspended in steam. The steam may be superheated. Alkali is added by impregnation, as the dry form, or in the form of a solution or slurry. The mixture so resulting is heated and given residence time in a pretreatment section.

Alternately, a pulverized coal-water slurry or coal-water-alkali slurry is used as the starting feed mixture. This material is heated and the water vaporized in a vaporizer section. As vaporization proceeds, the steam entrains the coal (and alkali) to produce a mix of coal (and alkali) suspended in steam. This then passes to the pretreatment section.

The alkali serves to capture sulfur compounds liberated during the pyrolytic decomposition which ensues, at the same time enhancing reaction between the steam and coal or othe carbonaceous materials. Control of relative feed rates, residence time and temperature at this pretreatment stage is necessary to the subsequent processing. The working pressure of the system is affixed at the condition best suited for equipment operability and for the other constraints required.

Two principal processes then follow as alternatives:

1. In what is referred to as single-step or direct conversion, the reacting mixture leaving the pretreatment section is passed up into and through an ebullating (fluidized) bed of methanation or Fischer-Tropsch type catalyst(s). Conversion is to methane, higher hydrocarbons, and/or oxygenated compounds, depending upon catalysts and conditions.

Carbon dioxide is the main coproduct, though minor amounts of hydrogen most usually will appear, as well as minor amounts of carbon monoxide, again depending upon catalysts, activity, and conditions.

The reactor proper is essentially adiabatic, though heat can, of course, be added or removed.

Control of temperature and residence time in the pretreatment section is essential to the control of the reactor temperature and conversion. This is in part because partial conversion to hydrogen and carbon monoxide tends to occur in the pretreatment section -- which is endothermic and absorbs heat. Whereas the overall conversion is essentially thermally neutral or autothermal, the further reaction of hydrogen and carbon monoxide is exothermic, giving off heat. Hence, the need for coordination between

0112117

operating the pretreatment section and the reactor proper.

At higher steam rates, hydrogen is alternately produced along with carbon dioxide, with appropriate temperature levels and catalysts -- e.g., iron oxides, but also with other methanation or Fischer-Tropsch type catalysts, even with alumina and silica compounds, and with alkalies alone.

The residence time, at temperature, of the reactor product stream is minimized after leaving the confines of the expanded catalyst bed. This is done by eliminating holdup space and by using a quenchcooler, direct or indirect.

A solids removal system (cyclone(s), filter, and/or baghouse) followed by a cooler-condenser completes the sequence.

2. In two-step conversion, the effluent from the pretreatment section is given additional residence time, if necessary, in an additional holdup vessel. This will depend upon the extent of the pretreatment.

Additional sulfur capture will occur in the holdup vessel, and additional alkali may be injected at this point, either dry or in the form of a solution or slurry.

The hydrogen/carbon monoxide/carbon dioxide ratios may be controlled in the pretreatment section, in the holdup vessel, and/or in a subsequent backup vessel by the regulation of relative reactant rates, temperatures (and pressures).

If the backup vessel is used, it can be charged with iron oxides or other CO-shift catalysts, to regulate further the $H_2/CO/CO_2$ ratios. If a fixed bed or catalyst is used, a cyclone or other solidsremoval system must be used ahead of the catalyst bed. If an ebullating (fluidized) bed is used, solids removal does not necessarily have to occur ahead of the bed.

The backup vessel, using iron oxides or similar materials, will also serve to capture any remaining sulfur compounds. A solids/removal system and cooler-condenser follow.

Further conversion with appropriate methanation or Fischer-Tropsch type catalysts constitutes the second step. The catalyst bed may be fixed or ebullating (fluidized). An ebullating bed does not necessarily require solids removal ahead of the reactor.

The reactor products are methane, higher hydrocarbons and/or oxygenated compounds, depending upon the catalysts and the conditions. The reactions

are highly exothermic, giving off heat, and requiring either interstage heat removal, cold product gas recirculation, or intrastage heat transfer.

Some conversion may occur in the backup vessel, particularly if an iron oxide is used for cleanup. Furthermore, the cooled gases from the backup vessel may not require reheat before entering the reactor for methanation or Fischer-Tropsch conversion. For some reaction will take place even at lower temperatures, causing a heating of the mixture due to the exothermicity, which further accelerates the conversion.

At higher steam rates, the first step may be directed toward hydrogen and carbon dioxide during pretreatment, at the holdup vessel, and/or at the backup vessel or even at the second step for that matter.

Furthermore, hydrogen/carbon dioxide mixtures also catalytically convert to methane, higher hydrocarbons and/or oxygenated compounds -- similarly to hydrogen/carbon monoxide mixtures. The presence of steam also plays a part in the stoichiometry of the reaction(s), via the CO-shift, and is no doubt a contributing factor along with the other reactant concentrations and the operating conditions and catalysts.

Therefore, the second-step reacting mixture in general is composed of hydrogen and both carbon monoxide and carbon dioxide, as well as water vapor and other minor constitutents. Pre-removal of the carbon dioxide is not necessary. In fact, cooling and condensation to remove the excess water (that is, steam) ahead of the second step is not absolutely necessary, depending upon the course of the conversion(s). Neither are the component proportions necessarily critical, since catalyst is more specific to the conversion. Nor is temperature necessarily critical within a range. The same applies for pressure; molar contractions, however, are favored by pressure.

Excess steam is used to drive the conversion. Upon cooling the products below the dew point, there results an aqueous condensate phase. This condensate phase will contain soluble byproducts, including ammonia and nitrogen compounds, soluble sulfur compounds, and even some carbon dioxide and hydrogen sulfide. These compounds will in part further react or ionize in solution.

Additionally, the condensate phase will contain dissolved or partially dissolved alkali carryover, including the portions that have become sulfided.

Finally, ash or ash-char particulate carryover will appear, along with carryover from the breakup or ablation of methanation or Fischer-Tropsch type catalysts.

The entire solids-free aqueous condensate phase can in fact be recycled to the steam generator or boiler provided the alkali does not cause fouling of the tubes during heating and change of phase. This will depend upon the design and operation. It is particularly apt for slurry feed.

In all cases it is preferable that the dry alkali does not reach a temperature where it will flux with metal surfaces or ash components.

Any oily phase or phases produced -- as the result of pyrolysis carryover and/or Fischer-Tropsch type catalyst -- can also be recycled, to the original feed, to the pretreatment section, and/or to the reactor(s), or to holdup and/or backup.

Unreacted or partially unreacted coal or char-ash can be recycled as dry or wet solids, to the feed, to the pretreatment section, or to the reactor(s). Or to holdup or even backup.

The activity of the methanation or Fischer-Tropsch type catalysts is controlled in two ways:

By the injection or recycle of steam, carbon dioxide and/or other oxidizing agents.

Alternately, by the injection or recycle of hydrogen and/or carbon monoxide or other

The preferable method depends upon the operating conditions, reactants and proportions, and catalysts. It cannot necessarily be predicted in advance. Care must also be exercised, so as not to deactivate the catalyst, especially at startup.

Control is maintained by injection in place, or by use of a separate regenerator vessel.

Activation procedures control both catalyst sulfiding and the degree of oxidation or reduction, both of which are related to catalyst activity -- in fact, determine catalyst activity.

The use of sulfur-resistant catalysts, such as molybdenum or tungsten compounds, notably the sulfides, minimizes the need for sulfur control.

Moreover, operating at higher temperatures, notably 1200-1500°F, reduces catalyst sulfiding and its effect on activity.

Ordinarily it is required that the methanation or Fischer-Tropsch catalyst be activated in place prior to startup of the feed streams. While catalyst may be purchased pre-activated, it is usually purchased in the oxidized state, requiring partial

reduction with hydrogen and/or carbon monoxide reducing agents. In some instances, if the catalyst is in a more metallic state, partial oxidation with oxygen, steam, and/or carbon dioxide as oxidizing agents is required.

It should be noted, however, that the catalyst will tend to activate after startup due principally to the presence of the above-mentioned gases, notably hydrogen (and carbon monoxide).

A degree of carryover of the methanation or Fischer-Tropsch catalysts will occur due to ablation or breakup. This is more pronounced initially, with a fresh charge. This carryover may be separated from the ash by magnetic/paramagnetic methods. That is, the principal catalyst components are either magnetic or paramagnetic, and in some cases non-magnetic, and yield to this means of separation. The magnetic susceptibility is enhanced by heat treatment.

Otherwise, still other methods may be employed such as used in extractive metallurgy. These involve dissolution in acidic or basic media, followed by oxidation or reduction or other chemical changes, finally to yield precipitation of the catalyst components. Further activation is then usually required.

Beyond this are the several means of mineral

recovery used in mining such as classification or flotation.

Finally, the ash-catalyst mixture can be sent to the appropriate metals refining operation.

The degree of efficacy and the advisability of the above will depend upon the degree of breakup and carryover. It is expected that in most instances it will be cheaper to dispose of the catalyst carryover with the ash.

In the foregoing operations, alkali is carried through the system along with the ash or ash-char particulates, and is collected dry and also in the form of a slurry or solution from the condensation of excess steam. The dry alkali in turn may be leached to form a combined slurry or solution. This slurry or solution will contain sulfided alkali as the result of the capture of sulfur compounds. The mixture is subjected to stripping with steam and carbon monoxide. The sulfided portion is thereby regenerated. Recycle follows as a solution or slurry, or by more complete evaporation to the dry alkali solid.

The aqueous phase(s), in whole or in part, may in fact be recycled to the steam generator or boiler provided the alkali or other components present do not cause fouling of the tubes during evaporation. This will depend upon design and pretreatment.

Recycle is particularly apt for slurry feed.

Any oily phase or phases produced -- as the result of pyrolysis or Fisher-Tropsch type reactions, or other reactions -- can also be recycled: to the original feed, to the pretreatment section, or to the reactor(s).

Alternately, the oily phase itself becomes a product stream, for fuel or for refining. Aromatic and cyclic components which may be present contribute to its value as a motor fuel. Oxygenated compounds which may be present, such as alcohols, may also contribute to motor fuel properties, notably the octane rating.

Unreacted coal or partially unreacted coal or char-ash can be recycled in part as dry or wet solids: to the feed, to the pretreatment section, or to the reactor(s). This recycle may also include catalyst(s) carryover.

The Drawings

Figure 1 is a schematic illustration of the process diagram and equipment for practicing the invention;

Figures 2(a) and 2(b) are illustrations in schematic form of alternative feed mechanisms for the process; and

Figure 3 is an illustration of a modified form of the present invention utilizing an additional step in the process.

DESCRIPTION OF THE PRESENT INVENTION

Particles of coal or other carbonaceous material are suspended in steam, or otherwise mixed with steam, in the presence of alkalies or alkali carbonates, chiefly sodium carbonate or potassium carbonate, but preferably the former. While both have a catalytic action on the steam-gasification of coal, sodium carbonate is more receptive to reaction with hydrogen sulfide. Carbonate ores, such as trona and nahcolite, may also be used.

The alkali carbonates of lithium and cesium are also active, as are the hydroxides (and oxides) of all the aforementioned elements, as are still other compounds. The more reasonable cost of sodium carbonate or its ores, along with its activity to the capture of hydrogen sulfide, make it the preferable choice.

The coal may be injected into the steam, or a coal/water slurry may be subjected to vaporization, cause the coal particles to become entrained or suspended in the steam so produced.

The alkali may be introduced dry, as a water

slurry, or impregnated upon the coal particles (from solution or slurry).

The coal particle size may range from a coarse mesh size down to the pulverized form, but preferably the latter. Even micron sizes may be used.

The coal/steam (or coal/water) proportions may vary widely. Ideally, the proportions are from about 50/50 on a weight basis to a mixture containing 50% or more water (or steam) than coal. The proportions will also depend upon the final use intended.

While the discussion here is confined to coal, it is intended that any carbonaceous material, both liquids and solids, can be used.

PRETREATMENT

The ensuing mixture of coal/water/alkali is heated to temperatures at which significant conversions occur. These temperatures are in the range of 1200-1500°F even higher, but preferably to around 1300-1400°F. Heating is accomplished via indirect heat transfer, as embodied in a shell-and-tube heat exchanger, tube-and-tube heat exchanger, tubestill or other heat transfer device. The heat content of the steam used -- which may be superheated -- can also contribute. In fact the original steam temperature can be in excess of the aforesaid temperatures

(1200-1500°F), achievable by the use of high-temperature heat transfer devices such as fired ceramic checkers or firebricks, used directly or indirectly, or other porous structures and devices. The injection of coal and of alkali (e.g., slurries) will then lower the mixture temperature to the acceptable range.

It is desirable and probably even necessary, moreover, to operate at all times below the fluxing point or melting point of the alkali. (The melting point of sodium carbonate is 851°C or 1564°F.) This will lessen the probability of particle agglomeration, and possibility of fluxing with equipment surfaces.

The heated mixture, at temperature, is allowed sufficient residence time, holdup time or soaking time from 10 seconds to one hour to produce a pyrolysis and thermal release of hydrogen sulfide and other volatiles. At these temperatures, particularly in the presence of sodium carbonate, the hydrogen sulfide is captured by the dry alkali, either as alkali impregnated upon the coal particle or as entrained alkali particles. Molten or fluxed alkali will also capture hydrogen sulfide, in the event this condition is reached. (The preceding is to be distinguished from the action of hydrogen sulfide in

the presence of aqueous alkali carbonate solution. In the latter, at elevated temperatures, e.g. boiling or near boiling, the hydrogen sulfide will be stripped from the solution -- that is, will not be absorbed, particularly in the presence of carbon dioxide.)

At the same time, there is the endothermic reaction of coal and steam occurring, resulting in the formation chiefly of carbon monoxide and hydrogen (but also of carbon dioxide). This reaction absorbs heat and causes the temperature to drop unless additional heat is added, a purpose of using heat transfer.

In further explanation, the reaction of carbon or coal with steam to form carbon monoxide and hydrogen is highly endothermic -- absorbs heat or requires heat to sustain the reaction. Other reactions also occur which may be either endothermic or exothermic -- that is, may either absorb heat or give off heat --notably the pyrolytic decomposition of the coal. Also occurring to a degree is the carbon monoxide-shift or CO-shift reaction, between carbon monoxide and steam to form carbon dioxide and hydrogen, and which is favored by lower temperatures (circa 1000-1200°F).

The juxtaposition of residence times, the heat added, the temperature and pressure, and other conditions and controls will in turn determine the outlet composition and conditions.

The aforesaid operations are regarded as the pretreatment step.

Reactions

Representative reactions occurring during the pretreatment step are as follows:

Conversion of the coal:

$C + H_2O(g) \rightarrow CO + H_2$ (Water-gas reaction; highly endothermic)...(18)

$CO + H_2O(g) \rightarrow CO_2 + H_2$ (CO-shift; moderately exothermic) ....(19)

Sulfur capture:

$H_2S + Na_2CO_3 \longrightarrow Na_2S + H_2O(g) + CO_2$ ...(20)

DIRECT CONVERSION

The resulting reacting mixture, after the prescribed pretreatment, may be injected into the bottom of a fluidized or ebullating bed of methanation or Fischer-Tropsch type catalyst, where it passes up through and out of the bed, being further converted to methane or heavier hydrocarbons, and/or oxygen compounds (e.g., alcohols), in the process. Ideally, the aforementioned conversion is near-autothermal (does not require the addition or removal

of heat). The term thermally neutral also applies.

The products are further processed and separated into the respective component streams by methods described elsewhere.

Included is quench/cooling of the overhead off-gases to prevent reversion of the products, followed by solids separation, cooling and partial condensation of the gases, and acid gas removal and recovery. There are also provisions for alkali catalyst regeneration and sulfur recovery, and for regeneration of the methanation or Fischer-Tropsch type catalysts.

The quench/cooler can be operated either by direct or indirect heat transfer. For the former case, either liquid water or low-temperature steam may be used. Unfortunately, additional concentrations of steam (or water) may cause the product composition to change unless a very rapid quench is achieved. An oil may also be used.

The methanation or Fischer-Tropsch type catalysts are for the most part oxides of the Group VIII transition metals, chiefly nickel, cobalt and iron. Activation occurs by adding reducing agents such as hydrogen and/or carbon monoxide prior to startup, at temperatures around 700-800°F. Self-activation can also occur, since hydrogen and carbon monoxide are

present or may be produced initially, or may occur as reaction intermediates.

Tungsten and molybdenum compounds -- e.g., the sulfides -- are also active, particularly to methane formation. The sulfide forms, especially, are resistant to sulfur poisoning.

Zinc oxide-based catalysts, another special case of Fischer-Tropsch type catalysts, are selective to methanol production.

Representative temperatures are 1200-1400°F and pressures of atmospheric to 1,000 psi.

Any further hydrogen sulfide released from the coal tends to be captured either or both by the alkali carbonate or by the metallic oxide catalysts. In the former case, no problem is presented. In the latter, the activity of the catalyst may be somewhat impaired, though this is not serious at the higher-than-ordinary operating temperatures used. Furthermore, in the presence of hydrogen, particularly, the Group VIII oxide catalysts tend to remain active at these temperature levels.

However, catalyst sulfiding may also be counteracted in place by the addition of small amounts of oxygen (or air), either at the bottom of the reactor or up and down the reactor. The action of steam and

oxygen counters sulfiding. Carbon dioxide also has an effect.

Reactions

Representative reactions occurring during direct conversion are as follows, in idealized proportions:

Conversion of the coal:

$C + H_2O(g) \rightarrow CO + H_2$ Endothermic .....(21)

$\frac{1}{2}(CO + H_2O(g) \rightarrow CO_2 + H_2)$ Moderately exothermic.(22)

$\frac{1}{2}(CO + 3H_2 \rightarrow CH_4 + H_2O(g))$ Exothermic .....(23)

---

Overall: $C + H_2O(g) \rightarrow \frac{1}{2}CH_4 + \frac{1}{2}CO_2$ Thermally neutral ......(24)

The above utilizes a nickel co-catalyst, along with the alkali, to yield methane ($CH_4$). With iron co-catalysts, for instance, hydrocarbon liquids are also produced. The production of alcohols is also a possibliity, using iron, zinc or other catalysts, and at appropriate conditions.

Steam in the proportions roughly of 50% excess is required to drive the conversion.

Sulfur capture:

$H_2S + Na_2CO_3 \rightarrow Na_2S + H_2O(g) + CO_2$ .....(25)

$H_2S + NiO \rightarrow NiS + H_2O(g)$ .....(26)

Regeneration:

$Na_2S + H_2O(liq) + CO_2 \rightarrow Na_2CO_3 + H_2S$ (External)..(27)

$NiS + \frac{1}{2}O_2 \rightarrow NiO + S$ (oxidation) (Internal or external)....(28)

or

$NiS + H_2 \rightarrow Ni + H_2S$ (reduction)(Internal or external).....(29)

The elemental sulfur carried over may be recovered as liquid or solid, depending upon the condensation temperature.

Activation

$$NiO + H_2(\text{or } CO) \xrightarrow{\phantom{xxxx}} \text{mixture of Ni and higher and lower oxides} + H_2O \quad .....(30)$$

or

$$Ni + O_2 \xrightarrow{\phantom{xxxx}} \text{mixture of Ni and higher and lower oxides} \quad .....(31)$$

Other Compounds

Other compounds such as ammonia ($NH_3$) will appear, and may be dissolved in the condensate, appear as a separate phase, or be mixed with the gaseous products. The means of removal and recovery will be specific to the substance. Of note in minor concentrations are hydrogen cyanide, carbonyl sulfide and carbon disulfide, and which will also be affected by sulfur control.

The nature of the compounds appearing will depend upon to what degree the atmosphere or conditions tend to be oxidizing or reducing, and to the

nature and proportions of the reactants, as well as other reacting conditions.

TWO-STEP CONVERSION

Alternately, the reacting mixture leaving the pretreatment section may proceed to an additional vessel to sustain the residence time. Further reaction takes place, chiefly to carbon monoxide, hydrogen and carbon dioxide.

Any additional hydrogen sulfide liberated is captured by the excess alkali still present. Additional alkali may also be added to the reacting mixture, either as a slurry or as a dry powder. In fact, alkali pellets may be added to form a fluidized or ebullating bed in this vessel, with provisions for recycle and regeneration.

The heat necessary to sustain the further conversion of coal and steam to carbon monoxide and hydrogen -- which is a strongly endothermic reaction -- may be supplied by the reaction or combustion of injected oxygen, either at the bottom or at points along the vessel. Injection may by accompanied by steam through tuyeres for instance. This steam would be a part of the total requirements.

With sufficient heat transfer and residence time, conversion of the coal can largely be effected in the pretreatment section. This obviates the use

of the additional vessel, and especially eliminates a need for the injection of oxygen to supply heat (via combustion of part of the carbonaceous material or other reactants). Moreover, the downstream end of the pretreatment section, or the additional vessel, can be operated adiabatically so that the endothermicity tends to cool down the reaction (or product) mixture.

Hydrogen Production

By adding considerable excess steam, conversion may proceed all the way to hydrogen and carbon dioxide. This is abetted by the presence of the alkali and by use of other catalysts such as iron oxides or alumina/silica catalytic cracking type catalysts.

Alumina/silica catalysts, incidentally, are resistant to sulfur capture and poisoning.

Off-Gas Treatment

The off-gases -- chiefly carbon monoxide, hydrogen and carbon dioxide, in varying proportions, methane, hydrocarbons and/or oxygenated compounds -- are quenched/cooled to a representative 900-1000°F for ease in separating the solids via a cyclone separator. The solids are mainly ash, sodium sulfide and unreacted sodium carbonate, or other derivatives.

The sodium compounds for instance are regenerated and recycled by methods explained elsewhere. Included is a water wash, and regeneration with heat (or steam) involving stripping with carbon dioxide.

The quench/cooler may be operated by direct or indirect heat transfer. With the former mode, liquid water or low-temperature steam may be injected into the hot gases. This will cause some further conversion to hydrogen via the CO-shift reaction, and should be regarded as part of the total steam requirement. The quench/cooler precedes the cyclone separator. Quench/cooling may be disregarded if materials problems do not occur in the cyclone separator.

As an option, the hot gases leaving the cyclone separator are conducted to a second bed of iron oxide or other agents either or both to promote the CO-shift reaction.

$$CO + H_2O(g) \longrightarrow CO_2 + H_2 \qquad \text{.........(32)}$$

and to capture any remaining $H_2S$. Steam or carbon dioxide may be added to move the CO-shift reaction either to the right or left as a means of controlling the $CO/H_2/CO_2$ ratios.

The bed may be fixed or else fluidized or ebullating. The latter is preferred in case any fines are carried in the gas stream.

The sulfided iron catalyst may be regenerated continuously by adding oxygen or air along with the feed or at points along the reactor vessel. The elemental sulfur is recovered downstream.

Alternately, the sulfided iron oxide may be regenerated externally in a separate vessel, using air or oxygen at appropriate temperatures, circa 1000°F, which is about the same temperature for the $H_2S$ adsorption. A fluidized or ebullating system is required. For fixed-bed operations, two or more contactors must be used, and alternated between adsorption and regeneration.

Note also that the iron oxide catalyst may at the same time have a catalytic effect, not only for the CO-shift, but for the subsequent Fischer-Tropsch reactions, as explained subsequently.

The off-gases from the second reactor, or the off-gases from the first cyclone, are passed to a second cyclone separator in the event fines are present in the gas stream. Otherwise, this second cyclone is not necessary.

In any event, the gases are sent to a cooler-condenser for removal of the excess water, which would inhibit further methanation or Fischer-Tropsch type reactions. Any liquids produced would be condensed, to a degree. Any free sulfur present from

sulfide oxidation is recovered along with the aqueous condensate.

Sulfur, for comparison, melts at about 120°C or 248°F. (The boiling point is 444.6°C or 832.3°F.) The sp. gr. of the solid is 2.0.

Off-Gas Conversion

Following the separation of the condensate phase(s), and of free sulfur if present, the cooled gases are sent to a conversion section for conversion to methane, higher hydrocarbons and/or alcohols, or methanol, etc. -- depending upon catalysts and conditions.

The conversion reactions, called methanation or Fischer-Tropsch type reactions, are between carbon monoxide and hydrogen, and/or between carbon dioxide and hydrogen. Thus CO and $CO_2$ can both be present. It is not necessary to remove the $CO_2$ by acid gas treatment prior to conversion. In fact, the $CO_2$ can serve as an oxidation agent to control catalyst sulfiding, carbiding, and activity.

Depending upon the operating pressure, the temperature of condensation will be a representative 212°F (the boiling point of water) or higher. It will also be related to the presence of dissolved solids, liquids and gases. Higher temperatures will lower the induction time for the ensuing exothermic

reactions. Alternately, a preheat or waste-heat exchanger can be used.

The ensuing conversions are highly exothermic, causing the temperature to rise accordingly, unless heat removal is used. Temperatures of 700-800°F are representative, though in cases --especially for methanation -- the temperature may be allowed to rise to 1200-1400°F.

For this reason, interstage cooling is called for. Alternately, hot-gas (or cold-gas) recycle can be used, or the reactor itself can be fabricated in the form of a heat exchanger, and internal cooling used (such as to a boiling liquid).

The first-mentioned is viewed as more flexible, and either a fixed or a fluidized or ebullating bed may be used.

The waste heat from the cooling cycle(s) is preferably used to generate process steam, to be used as a component of the feed mixture and other purposes, in particular for subsequent acid gas removal and recovery.

Operating Pressures:

A common system operating pressure may be used, from atmospheric or near atmospheric pressures to 1,000 psi. However, the initial gasification is favored by lower pressures. The cooled off-gases

after separation following the cooler/condenser (at a representative 212°F or higher) may be compressed, however, for methanation, or Fischer-Tropsch conversion including methanol production. Representative pressures are 300-500 psi. Compression, moreover, will cause a temperature increase.

Lower conversion pressures, particularly for methanol production, will require extremely active catalysts. This is due in large part to the fact that during reaction a contraction in moles or gas volume occurs, which is favored by higher pressures and inhibited by lower pressures.

Reactions

Representative reactions involved include the following:

Gasification and overall conversion to hydrogen:

$C + H_2O(g) \rightarrow CO + H_2$ $\qquad$ $C + O_2 \rightarrow CO_2$ .......(33)

Oxidative support

$CO + H_2O(g) \rightarrow CO_2 + H_2$ $\qquad$ $C + \frac{1}{2}O_2 \rightarrow CO$ .......(34)

$C + 2H_2O(g) \rightarrow CO_2 + 2H_2$ .......(35)

Sulfur removal by alkali:

$H_2S + Na_2CO_3 \rightarrow Na_2S + H_2O(g) + CO_2$ .......(36)

Regeneration in aqueous phase:

$Na_2S + H_2O(liq) + CO_2 \rightarrow H_2S + Na_2CO_3$ .......(37)

$NaHS + H_2O(liq) + CO_2 \rightarrow H_2S + NaHCO_3$ .......(38)

<u>Sulfur removal by iron oxide</u>:

$3H_2S + Fe_2O_3 \longrightarrow Fe_2S_3 + 3H_2O(g)$ .......(39)

<u>Regeneration by gaseous phase</u>:

$Fe_2S_3 + 3/2\ O_2 \longrightarrow Fe_2O_3 + 35$ .......(40)

<u>Methanation or Fischer-Tropsch type reactions</u>:

Methane:

$CO + 3H_2 \longrightarrow Ch_4 + H_2O(g)$ .......(41)

Higher hydrocarbons:

$CO + 2H_2 \longrightarrow (CH_{2_}) + H_2O(g$ .......(42)

Higher alcohols:

$CO + 3/2\ H_2 \longrightarrow (CH_{2_})OH$ .......(43)

Methanol:

$CO + 2H_2 \longrightarrow CH_3OH$ .......(44)

Other oxygenated compounds may also result, depending upon catalysts and conditions.

Alternately, with carbon dioxide and hydrogen reactants,

Methane:

$CO + 4H_2 \longrightarrow CH_4 + 2H_2O(g)$ .......(45)

Higher hydrocarbons:

$CO_2 + 3H_2 \longrightarrow (CH_{2_}) + 2H_2O(g)$ .......(46)

Higher alcohols:

$CO_2 + 5/2\ H_2 \rightarrow (CH_{2_})OH + H_2O(g)$ .......(47)

Methanol:

$CO_2 + 3H_2 \longrightarrow CH_3OH + H_2O(g)$ .......(48)

In a manner of speaking, the CO-shift can be assumed to be involved, since it relates CO and $H_2O$ to $CO_2$ and $H_2$.

The methanation or Fischer-Tropsch type catalysts can be activated in place, which also constitutes a metallurgical reduction process. For example:

$$Ni_mO_n \xrightarrow{H_2} Ni + H_2O \qquad \ldots\ldots.(49)$$

$$Fe_2O_3 \xrightarrow{H_2} FeO \quad \text{or} \quad Fe + H_2O \qquad \ldots\ldots.(50)$$

Even sulfided catalysts may be so regenerated, or ores in the form of sulfides may be so reduced, e.g.,

$$NiS \xrightarrow{H_2} Ni + H_2S \qquad \ldots\ldots.(51)$$

$$FeS \xrightarrow{H_2} Fe + H_2S \qquad \ldots\ldots.(52)$$

<u>Methanation or Fischer-Tropsch Catalyst Carryover</u>

Methods are available for the alkali or acid extraction of the catalyst carryover (in solution), followed by regeneration by hydrogenation (and reconstitution).

<u>Reacting Systems</u>

Under apropriate reaction conditions, and in the presence of suitably active catalysts, coal or other carbonaceous materials and steam are converted directly to hydrocarbon (and oxygenated) gaseous and

liquid fuels. Also involved are reactor and bed configurations for assuring multiphase contact, and the means for controlling catalyst activity and the ensuing conversion.

Of concern are reactions and conversions which involve in one way or another Fischer-Tropsch type reactions whereby carbon monoxide and hydrogen are reactants or may be reaction intermediates:

$$CO + H_2 \longrightarrow C_mH_n + H_2O + CO_2 \qquad .......(53)$$

or

$$CO + H_2 \longrightarrow C_mH_nO_p + H_2O + CO_2 \qquad .......(54)$$

The above representation is not intended to be stoichiometric. Alternately, carbon dioxide may be appear as a reactant:

$$CO_2 + H_2 \longrightarrow C_mH_n + H_2O \qquad .......(55)$$

$$CO_2 + H_2 \longrightarrow C_mH_nO_p + H_2O \qquad .......(56)$$

The carbon monoxide and hydrogen are derivable from the reaction of coal or other carbonaceous materials (denoted as C) with steam ($H_2O(g)$):

$$C + H_2O(g) \longrightarrow CO + H_2 \qquad .......(57)$$

and their proportions controlled by additional steam via the CO-shift reaction:

$$CO + H_2O(g) \rightarrow CO_2 + H_2 \qquad .......(58)$$

The type and molecular weight of the compounds produced will depend upon the particular catalyst, its activity, and the reaction conditions. For

instance, nickel is selective to methane, cobalt catalysts also produce liquids, and iron catalysts more so. While the liquids may be chiefly hydrocarbons ($C_mH_n$), oxygenated compounds may also be produced ($C_mH_nO_p$).

Furthermore, the overall conversion may also occur in integrated systems, using an alkali co-catalyst along with the Fischer-Tropsch type catalyst, so that coal or other carbonaceous materials convert directly with steam to the desired products. This may be represented as:

$$C + H_2O(g) \longrightarrow C_mH_n + CO_2 \qquad .......(59)$$

$$C + H_2O(g) \longrightarrow C_mH_nO_p + CO_2 \qquad .......(60)$$

where carbon monoxide (CO) and hydrogen (H or $H_2$) may be viewed as the reaction intermediates.

The catalyst is usually used in the higher oxide form, and may exist in several degrees or states of oxidation. Activation is by reduction, ususlly with hydrogen and/or carbon monoxide.

For catalysts in the reduced or metallic state, activation is by oxidation, with oxygen or other oxidizing agents, even carbon dioxide.

For the purposes here, the following remarks pertain to the following features:

1. Multiple Contacting Systems
(Ebullating and Entrained Conditions)

2. Temperature Control
3. Alkali Catalyst Recovery and Regeneration
4. Control of Catalyst Sulfiding and Carbon Deposition
   (Recycle and Regeneration of Group VIII and Other Catalysts)
5. Recovery of Group VIII Catalysts
   (by Magnetic Methods)

MULTIPLE CONTACTING SYSTEMS

(EBULLATING AND ENTRAINED CONDITIONS)

In a multiphase ebullating bed, the bed proper consists of a collection of particles mostly of uniform size (and shape). In the expanded or ebullating condition, the particles constitute a stationary phase (or phases) with the individual particles in random motion.

The random motion of the stationary phase is assured by the upward flow of the entraining phase(s). The entraining phase in turn carries suspended solid or liquid particles up, through and out of the top of the stationary phase. The entrained particles themselves may constitute one or more distinct phases.

The entraining phase itself be a gas or a liquid or some combination, but most usually a gas.

Furthermore, the interaction between the entraining phase and the stationary phase will impose a degree of circulation, or recirculation, upon the random motion of the bed particles.

The random motion of the particles of the stationary phase may be referred to as "ebullating," and the bed in its expanded condition may be referred to also as "fluidized." Strictly speaking, however, particles in a fluid or fluidized bed have a pronounced size distribution, ranging from the very small, through intermediate sizes to the relatively large. In the expanded state, this size distribution will result in the capture of entrained particles, and is to be avoided here.

In a true ebullating bed, on the other hand, the uniform size of the bed particles will allow the particles of the entrained phase to pass up through and out of the expanded bed.

A classification is set up. The size and density of the ebullating particles is such that the expanded bed, as a whole, remains in a stationary or fixed position relative to the vertical axis of the reactor vessel. On the other hand, the particles of the entrained phase are of a size and density (smaller and/or less dense) such that they remain suspended in the entraining phase or medium and are

carried up through and out of the bed. The shape of the particles also influences the separation.

The entrained phase may be introduced into the entraining phase before it enters the vessel, or may be introduced directly into the vessel.

The particles present in the ebullating bed proper may be cycled in and out of the bed for regeneration, replenishment, or other purposes.

TEMPERATURE CONTROL

The temperature requirements for the overall conversion are confined to relatively narrow limits. At lower temperatures, the Boudouard reaction

$$CO_2+C=2CO \quad \ldots\ldots\ldots\ldots(61)$$

shifts to the left, causing the deposition of carbon on catalyst surfaces, which can adversely affect activity. This may be controlled, however, also by the methods discussed under Control of Catalyst Sulfiding and Carbon Deposition.

At higher temperatures -- while the rate of conversion is enchanced -- reforming of the products occurs, producing a reversion to carbon monoxide and hydrogen or otherwise preventing the formation of the desired products in the first place.

Accordingly, there is a relatively narrow band from about 1200°F to 1500°F whereby the conversion is optimized. The upper limit is increased by higher

operating pressures, which may run from atmospheric or near-atmospheric to 1000 psi or more.

Finally, the off-gas products from the reactor are favored by a quick quench which also prevents a reversion to CO and $H_2$ after the products leave the confines of the catalyst bed. The quench may be accomplished indirectly or directly, the latter using water or cooled recycled liquids. The proportions and temperature of the direct quench cooling water must be regulated in order to minimize steam reforming of the products.

Also, by controlling the temperature and residence time of the catalytic conversion, heavy oils and tars can be either minimized or eliminated, or else favored by the appropriate catalyst and conditions.

With nickel or simular methanation catalysts, the conversion is specific to methane, and no higher hydrocarbon or oxygenated compounds are produced (oils or tars) if sufficient residence time is provided.

## ALKALI CATALYST RECOVERY AND REGENERATION

In the conversion of coal or other carbonaceous materials by gasification, alkali carbonates, particularly, are used as a catalyst. At

the same time, the alkali picks up sulfur liberated chiefly as hydrogen sulfide under the reducing atmosphere and temperature of the conversion. This section pertains to the recovery and regeneration of the alkali so used, and at the same time to the retrieval of the sulfur, first as hydrogen sulfide.

The alkali, usually in the form of sodium or potassium carbonates, fluxes with the coal particle surface as it is heated, causing a breakdown or modification of the coal structure and enhancing the reactivity of the coal particle to steam, oxygen or other reactants during gasification.

Concurrently, the pyrolyis of the coal and the action of the alkali cause a liberation of sulfur as hydrogen sulfide principally, but also other forms, followed by sulfiding of the alkali. The product, e.g., sodium sulfide will exist in part along with unreacted alkali.

The ash or ash-char so resulting form the conversion will contain both free alkali and sulfided alkali. The separation is as follows:

The mixture is contacted with water, preferably hot water, which may be added or else occurs during the conversion (such as from excess steam). The free alkali and sulfided alkali dissolve in the

water phase, along with other solubles which may be present. The insolubles are separated out.

The solution containing the dissolved free alkali and sulfided alkali is contacted with carbon dioxide gas at elevated temperatures, near boiling. Free steam may in fact be added. This produces a reaction representable symbolically as:

$$Na_2S + CO_2 + H_2O \longrightarrow Na_2CO_3 + H_2S \quad .......(62)$$

the bisulfide, NaHS, may also occur and be regenerated.

The regenerated alkali remains in solution, and may be concentrated by evaporation before being recycled to the reacting system. Concentration may be carried out to form a slurry, or even to dry alkali.

Hydrogen sulfide and carbon dioxide used for the stripping will be the principal gaseous byproducts. One may be separated from the other by selective absorption (or adsorption) techniques. The degree of separation may be enhanced by the use of extractive reflux.

Alternately, the hydrogen sulfide/carbon dioxide gaseous mixture may be sent to a Claus unit, or its equivalent, for conversion to elemental sulfur.

Other water-soluble compounds in the condensate or aqueous solution mixture may require pretreatment or after-treatment. In the case of ammonia, heating the solution, which is basic, prior to contacting with carbon dioxide, will drive off the ammonia. Whether the nitrogen contained in the carbonaceous material appears as ammonia, molecular nitrogen, and/or other compounds depends upon the reaction conditions, particular the degree of reducing/oxidizing conditions -- e.g., the presence of hydrogen and carbon monoxide as reducing agents versus the presence of steam and carbon dioxide (and possible oxygen) as oxidizing agents.

Phenolic compounds and hydrogen cyanide, being acidic, will tend to remain in solution. Other oxygenated compounds will also tend to remain in solution. These oxygenated compounds may be separated during evaporation, or remain in the aqueous concentate. In either case they may be recycled to the reactor, along with the regenerated alkali, and be further converted in the gasifier.

The hydrogen cyanide will be driven off in the carbon dioxide contractor, along with the hydrogen sulfide (and excess carbon dioxide). It may be selectively removed and recovered by a water wash of

controlled pH, and ultimately converted to sodium or potassium cyanide, or converted to ammonia, etc.

Carbon dioxide recovered from the gaseous reactor products may in part be used for the stripping operation to regenerate the alkali and remove the $H_2S$ so produced.

CONTROL OF CATALYST SULFIDING AND CARBON DEPOSITION (RECYCLE AND REGENERATION OF GROUP VIII AND OTHER CATALYSTS)

Fischer-Tropsch type catalysts, which are mainly or Group VIII transition metals, are affected by sulfide poisoning. That is, the presence of hydrogen sulfide, particularly, is noted to reduce catalytic activity -- depending upon the operating conditions. This is true notably for nickel and to a lesser extent cobalt and iron.

Since coal, particularly, contains sulfur compounds which decompose or react to give off hydrogen sulfide under conversion conditions, and since hydrogen sulfide can adversely affect catalyst activity and conversion, a means of sulfide control or catalyst regeneration is desirable.

A method of control is to circulate the catalyst to a regenerator where it is contacted with steam and oxygen. This converts the sulfided catalyst back to the oxide form, which is the active form. The sequence for regeneration may be viewed as

follows, using a sulfided nickel catalyst as the example:

$$2NiS + 2\ H_2O(g) \rightarrow 2\ NiO + 2\ H_2S \qquad ......(63)$$

$$NiS + 3/2\ O_2 \longrightarrow NiO + SO_2 \qquad ......(64)$$

$$2\ H_2S + SO_2 \longrightarrow 2\ H_2O(g) + 3/2\ S_2(g) \qquad ......(65)$$

---

$$3\ NiS + 3/2\ H_2O(g) \rightarrow 3NiO + 2\ H_2O(g) + 3/2\ S_2(g).(66)$$

Suffice to say, oxygen in the presence of steam produces the reaction. The steam also controls the activity of the oxygen. However, steam itself can also act as an oxidizing agent.

Since the steam already occurs in the conversion mixture, there is the option of adding oxygen directly to the system to control the sulfiding -- thus avoiding a separate regenerator.

The regenerator off-gases may be sent to the sulfur recovery system.

Treatment of the catalyst with steam and oxygen also removes any carbon deposition on the catalyst. The carbon is converted principally to carbon monoxide (and hydrogen, via the CO-shift), which also serves to convert the sulfide to oxide, while at the same time activating the catalyst.

Alternately, the catalyst may be regenerated directly from $H_2$ and/or CO produced externally (as from the coal-steam or water-gas reactions):

$$C + H_2O(g) \longrightarrow CO + H_2 \qquad ......(67)$$

and

$$CO + H_2O(g) \longrightarrow CO_2 + H_2 \qquad ......(68)$$

Simply stated, the sulfided catalyst is in effect oxygen-blown to remove the sulfur (which is enhanced by the presence of moisture or steam), and is then reduced to the activated state. As an alternative, oxygen may be introduced in limited amounts to control catalyst activity without oxidizing the hydrocarbon or oxygenated products ordinarily obtained.

$$C + H_2O(g) \longrightarrow C_mH_n + CO_2 \qquad ......(69)$$

The object is to control catalyst activity without appreciably oxidizing the hydrocarbon product. The oxygen may be introduced in the pretreatment section, where it may also react with coal to provide a degree of superheat to the reactants. It may also be added directly to the reactor.

A catalyst may be recycled to a regenerator, or two or more reactor vessels may be used with cycling between reaction and regeneration. The concept also applies to fluidized (and/or ebullating beds) and to fixed beds for reacting carbon monoxide and hydrogen.

As an added note, carbon deposition may be controlled by the carbon dioxide level, or by the

recycle of carbon dioxide. The reaction involved is primarily the Boudouard reaction:

$CO_2 + C = 2\ CO$ .......(70)

This may be accomplished within the reactor proper or within the regenerator.

## PROCESSING DETAILS

Integration of the afore-described features is shown in the flow diagrams represented by Figure 1 and Figure 2, and a s described follows:

Direct Conversion:

Referring to Figure 1, pulverized or finely-divided carbonaceous materials (e.g., coal) and alkali (e.g., sodium carbonate) are injected into steam by a conventional mixer 10 and supplied via a conduit or stream 11 to a valve 12. The alkali may be impregnated upon the carbonaceous material, if desired, or powdered or granulated alkali may be mixed with the carbonaceous material or introduced separately, dry or as a solution or slurry.

Alternately, the pulverized or finely divided carbonaceous material, plus alkali, is mixed with water in a mixer 13 to form a slurry and supplied via a conduit 14 to a vaporizer 15 and the valve 16. Hydropulping or similar methods can be used to divide and suspend more fibrous materials. The slurry then passes through a vaporizer 15.

Either valve 12 or 16 may be opened to use one of the material supplies in the result. The result, in either case above, is finely-divided carbonaceous material and alkali entrained or suspended in steam which comprises a stream 17. Stream 17 is formed at essentially reactor system pressure and is exclusive of air.

Stream 17 is passed through a pretreatment section 18, where it is heated to approximately reactor temperature (circa 1300°F). This will depend upon the degree of pre-reaction occurring to form $H_2$, CO, and $CO_2$. Additionally, unreacted char-ash (as will be explained hereafter) may be introduced for recycling from an input 19. Also oxygen or other oxidizing agents may be added via a conduit 20. Reducing agents such as hydrogen or carbon monoxide can also be supplied via conduit 20. These assist in controlling the degree and course of pre-reaction, along with temperature. Temperatures, in all cases, are preferably kept below the melting point of the alkali (1564°F for sodium carbonate).

The exit mixture from the pretreatment section designated stream 21, is injected into the multiphase reactor-contactor 22. Recycled car-ash from input 19 may also be introduced into the multiphase reactor-contactor 22, as well as oxidizing or reducing agents

from conduit 20. Additional alkali may also be introduced at this point.

The principal features of the pretreatment section are diagrammed in Figures 2(a) and 2(b). In the first version shown in Figure 2(a), coal/alkali particles suspended in steam flow through a direct-fired tubestill heater 23, or other type of heater, and are injected into the bottom of the reactor or other vessel 24. The tubestill heater 23 may be fired by a part of th total feedstock used, by char product, by gaseous or liquid products or by other fuel sources.

Alternately, as shown in Figure 2(b), the feedstock may be introduced and carried through the pretreatment section 18a by means of a screw or auger, rotating inside a heated tube 18a. The tube 18a is heated externally by direct firing of a suitable fuel or by other means.

To this augered feed may be added steam, alkali, or other catalyst. The coal may be injected into the side of the reactor 26 preferrably near the bottom, and superheated steam in total or in part may be injected by a conduit 27 from the bottom (as through a perforated plate). Part or all of the alkali may be introduced with the steam at the bottom. In addition to providing an alternate means for

reactor operation, it will provide an additional means for controlling the gaseous $H_2/CO/CO_2$, ratio entering the reactor.

The multiphase reactor-contactor 22 operates with an expanded bed of ebullating (fluidized) methanation or Fischer-Tropsch type catalyst particles. The catalyst particles are approximately uniform in size and shape. Typical sizes are 1/8" or smaller, to 1/4" or larger. Spherical or cylinder shapes, or other shapes, may be used -- preferably the former to minimize breakup and "rounding off of the corners." After an initial induction period, a catalyst of suitable mechanical strength (e.g., with a ceramic substrate) will level off to only small attrition rates. The bed is further characterized as a "fixed" or "stationary" ebullating bed.

The coal/alkali/steam reacting mixture passes up through and out of the ebullating bed. Properly speaking, this is what distinguishes an "ebullating" bed of uniform particles from a "fluidized" bed, which has a size distribution and will tend to capture other particles within the size range. The terms are often used interchangeably, however. Reactor residence time after leaving the bed is minimized by reducing or eliminating holdup space between the top of the expanded bed and the outlet.

Depending upon needs, the catalyst from the multiphase reactor-contactor 22 is circulated to a regenerator 28. There the catalyst is contacted as an ebullating (fluidized) bed with oxidizing or reducing agents via conduit 20. The regenerator 28 is preferentially at essentially system pressure. The regenerator 28 is preferably adiabatic with the catalyst introduced at approximately the temperature at the multiphase reactor-contactor 22. The oxidizing or reducing agent from conduit 20 may be introduced at a lower temperature, or may be heated (or cooled). The regeneration reactions are expectedly exothermic or endothermic, depending. The regenerator 28 may be used to activate the catalyst at startup. Stream 30, the off-gases from the regenerator 28, and which will contain sulfur oxides and/or sulfur, or hydrogen sulfide, depending, is sent to gas cleanup. This may comprise a separate section, but preferably will involve sulfur conversion and hydrogen sulfide recovery units used elsewhere.

Stream 31 from the contactor 22 comprises the effluent product mixture of gases and entrained solids leaving the multiphase reactor-contactor 22. Stream 31, with minimal residence time, may first be

passed through a quench means 32, to lower the temperature and prevent further reaction, reforming, or regression of the product. Typical quench temperatures are 900°F, plus or minus. The quench means 32 may be direct, with the injection of water or oil. Or indirect heat transfer can be used to heat cooling water, steam, or other media.

The effluent mixture from the quench means 32 is then passed to a solids separation system 34, which may consist of a cyclone separator, a filter, electrostatic precipitator, and/or a baghouse (for appropriate operating temperatures). Most of the solids are collected here, depending upon separation efficiency. The separated solids are output to the conduit 19, part of which may be recycled to preatment section 18 and/or to the multiphase reactor-contactor 22. The remainder or bulk of the output of the solids separator system 34 which consists of ash or char-ash, alkali and sulfided alkali, and any catalyst carryover, is sent to a mixer-contactor 35 for alkali recovery. The gaseous effluent output 36, is passed through a cooler-condenser 37 and on to a separator or receiver 38. Lower separator temperatures are preferable, circa 100-120°F, in order to retain ammonia and other volatiles in the aqueous

phase, and to retain volatiles in any oily phase produced. Lower temperatures also favor retention of sulfided alkali in the presence of carbon dioxide.

The gas product from the separator 38 will consist primarily of methane and other volatile hydrocarbon and/or oxygenated compounds, hydrogen, and carbon dioxide, depending upon catalysts and operating conditions. At higher reactor temperatures, carbon monoxide may show. Hydrogen sulfide may also show, depending upon the alkali used and its effectiveness. The gas product may be used as is or sent to acid gas cleanup for removal of carbon dioxide (and any hydrogen sulfide) and dehydration.

Depending upon catalysts and conditions an oily phase may or may not be produced at the separator 38. This may be recycled in whole or in part to the multiphase reactor-contactor 22. Any net produced may be used as is for fuel or other purposes, or be further refined.

The aqueous phase and sludge from ash or ash-char and catalyst carryover, is output from the separator 38 via conduit 40 and will also contain alkali and sulfided alkali carryover, plus any free sulfur which may appear from oxidizing-reducing reactions. The output 40 is heated, under pressure, to

about 250°F, the melting point of sulfur. The heated mixture is flashed or otherwise stripped (with steam) to produce ammonia and other volatiles as the off-gas, which is sent to processing. The liquid underflow product is liquid sulfur, for washing and recovery. The aqueous overflow is sent to the mixer-contactor 36 along with the sludge. Flotation methods, or other techniques, can also be used to separate the sulfur.

The underflow from the mixer-contactor 35 consists of a sludge of ash or ash-char plus any catalyst carryover. It is sent to disposal, or to separation and recovery of the catalyst components, along with regeneration and/or reconstitution.

The overflow output 43 from the mixer-contactor 35, consists of an aqueous phase containg dissolved alkali and sulfided alkali. This is sent to a contactor 41, where it is preheated and/or heated with steam, directly or indirectly. Carbon dioxide is at the same time injected, acting to regenerate the sulfided alkali and to produce hydrogen sulfide and other liberated components. The off-gases, therefore consist essentially of hydrogen sulfide and excess carbon dioxide (and steam). These are labeled acid gases. These gases are sent to a

Claus type unit, or other unit, for conversion to elemental sulfur.

The bottom output stream from the contactor 41 is an alkali solution or slurry. It is either recycled to steam generation or to the slurry feed system, or else concentrated for an alkali slurry injection, or evaporated for dry injection.

Two-Step:

Referring to Figure 3, the alternate feed systems are the same as in Figure 1. The resulting entrained feed mixture is again introduced into the pretreatment section via conduit 17. Recycle from conduit 19, the solids from solids separator 34, may be introduced into the pretreatment section 18, as well as oxidizing or reducing agents via conduit 20. The degree of sulfur capture and conversion again depends upon the heat added, the conditions, and recycle and injection. For the purposes here, a high degree of conversion is sought in pretreatment section 18. The proportions of carbonaceous material and steam will also determine the $H_2/CO/CO_2$ ratio achieved. Outlet temperature levels are again maintained at circa 1300°F. Fresh or recycled catalyst may be introduced ahead of pretreatment section 18. Also carbon monoxide, carbon dioxide, or other reactants or recycled products may also be injected, as

mentioned.

The principal features of pretreatment section 18 are shown in Figure 2, and apply here as well. The coal/alkali/steam entranced mixture may be heated in a fired heater, and injected into the succeeding vessel, in this case the holdup section.

Alternately the coal may be fed and moved through the pretreatment section 18 by means of a screw or auger, as previously described. Superheated steam and alkali may also move simultaneously with the coal, or may in whole or in part be added after pretreatment. This will also serve to control the gaseous $H_2/CO/CO_2$ ratio.

The effluent output 21 from pretreatment section 18 may or may not be sent to a holdup section 45. This is a vessel provided to achieve further residence time for sulfur conversion and reaction. Solids recycle from conduit 19 may be introduced, and additional alkali injected, as well as oxidizing or reducing agents via conduit 20. Heat may also be added (or removed) at holdup section 45. The temperature is affected by further conversion reactions occurring, and by the effect of oxidizing or reducing agents. All things considered, the temperature level can be allowed to fall to circa 700-900°F, but also higher or lower.

The effluent ouput 46 from holdup section, is passed through solids separator 34, which consists of a cyclone separator, filter, electrostatic precipitator, and/or baghouse suitable for the temperature levels encountered. Most of the solids are collected here, depending upon separation efficiency. The output 19 for the solids removed, may in part be recycled to pretreatment section 18 and/or holdup section 45. The net is sent to the mixer-contactor 47.

The overhead gas from separator 34, may or may not be passed through a backup section 50. This may be provided to achieve still additional residence time, but more importantly can provide additional sulfur capture by employing iron oxide pellets or other agents. The bed may be fixed or ebullating (fluidized). Additionally, Fischer-Tropsch type reactions may occur due to the catalytic nature of the iron oxide or other agents. These reactions may increase the temperature. Levels of 700-900°F, but also lower or higher, are incurred.

The effluent output from backup section 50, is again subjected to a solids separator 51. Part of the solids removed, stream 52, may be recycled to backup section 50 (or elsewhere, e.g., pretreatment section and/or holdup section). The net is sent to

the mixer-contactor 47. With fixed-bed operation, solids carryover is negligible. However, this will also depend upon solids carryover from separator 34. With appreciable solids carryover from separator 34, backup 50 will require an ebullating bed.

The overhead output from separator 51, is passed through a cooler-condenser 54, then to a separator 55. Lower temperatures favor the retention of ammonia and other volatiles, and the retention of sulfided alkali in the presence of carbon dioxide. Depending upon prior conditions an oily phase may appear which can be used as fuel, or further processed; or recycled (e.g., to pretreatment section). The bottoms of the separator 55 will contain dissolved gases, any further alkali and ash or ash-char carryover, and any free sulfur liberated due to oxidizing-reducing reactions. This stream is heated under pressure to 250°F, then flashed (and stripped with steam if necessary). The off-gases from flashing and stripping will contain ammonia and other volatiles. Liquid sulfur is withdrawn as a side steam, for washing and recovery. The aqueous overflow, containing any dissolved alkali or sulfided alkali carryover, is sent to the mixer-contactor 47. Any sludge from ash or ash-char carryover is removed as underflow and sent to the mixer-contactor 47.

Floation, or other techniques can also be used to separate the sulfur.

The gaseous overhead from separator 55 is sent to a Fischer-Tropsch or F-T reactor 57. With lower temperatures in separator 55, in order to retain volatiles, it may be necessary to heat the gaseous overhead. The principal components of the gaseous overhead carbon monoxide, and carbon dioxide. The makeup will depend upon the mix of the input, and the subsequent processing conditions. This is all tailored to the catalyst used and the desired final product mix.

The methanation or Fischer-Tropsch reactions occurring in the F-T reactor 57 are endothermic, requiring temperature control. The F-T reactor 57 may be sketched in sections to facilitate interstage heat removal or intrastage heat removal. Heat transfer may be to water, boiling water or steam, or to other media such as dowtherm. Cold gas recycle can also be used, injected at an appropriate points(s).

The bed in the F-T reactor 57 may be fixed or ebullating (fluidized). With the latter it is advisable to use a solids separator 58 on the output stream 11, the F-T reactors 57 effluent. Any solid catalyst carryover may be recycled (stream 59).

Stream 60, the overhead from separator 58, is sent to a cooler-condenser 62, and then to a separator 63. Depending upon conditions an oily phase may appear, which may be used as fuel or further processed, or recycled (e.g., to pretreatment section).

Stream 64, which represents any aqueous phase produced, may be used in the mixer-contactor 47, or recycled to feed preparation for steam generation or slurry makeup. If valuable water-soluble components occur, such as alcohols, stream 14 may be further processed. As a particular case, in the production of methanol, water may be produced only as a minor constituent.

The gas product from separator 63 will contain non-condensable gases produced, depending upon the feed composition to the F-T reactor 57, the catalyst used, and temperature and pressure. Gases appearing include methane and/or volatile hydrocarbons and/or volatile oxygenated compounds, plus carbon dioxide and water vapor. The gas product may be in part recycled to the F-T reactor 57 as cold gas recycle. The remainder is sent to gas cleanup and dehydration.

The underflow from the mixer-contactor 47 will consist of ash or ash-char plus any catalyst carryover. It is sent to disposal. If catalyst components are appreciable, reclamation procedures can

be used.

Stream 70, the overflow from the mixer contactor 47 is composed of dissolved alkali and sulfided alkali. Stream 70 is sent to a contactor 71. Preheat can be used. Steam or heat is introduced at the contactor along with carbon dioxide. The carbon dioxide (and steam) regenerates the sulfided alkali, and strips the hydrogen sulfide found. The off-gases, designated acid gases, etc., are sent to sulfur recovery, for removal of $H_2S$ and conversion to elemental sulfur via a Claus or other unit.

The bottoms from the contactor 70 is again labeled alkali solution or slurry. It is either recycled to steam generation or to the slurry feed system, or else concentrated for alkali slurry injection, or evaporated for dry injection.

## SUMMARY

This invention pertains to the catalyzed conversion of coal or other carbonaceous materials with steam, as previously outlined. The chief products variously include methane, higher hydrocarbons, hydrogen and oxygenat compounds plus carbon dioxide coproducts. Other products such as sulfur and ammonia may also ensue. Means are provided for substantially reducing this invention to practice by controlling sulfur and catalyst activity and by detailing other processing arrangements. The principal features are:

1. Control of steam rate (steam/coal ratio)

Intermediate rates favor methane

Theoretical: Approximately 1/1 on weight bases

Actual: About 50% over theoretical

Higher rates favor hydrogen:

Theoretical: Greater than approximately 1/1

Actual: About 50% over theoretical

Lower rates favor higher hydrocarbons and/or oxygenated compounds

Theoretical: Less than approximately 1/1

Actual: About 50% over theoretical

2. Injection of reactants

Dry or moist coal or other carbonaceous materials are injected into wet, dry or superheated steam. These other carbonaceous materials may include lignite, peat, municipal or agricultural organic wastes, rubber (tires), wood waste, chars, oils, etc.

Superheated steam can be used to contribute to the enthalpic energy of the steam

Coal or other carbonaceous materials are mixed with water to form a slurry. Alkali can be added to the slurry.

For fibrous materials, hydropulping can be used.

Alternately, the coal or other carbonaceous materials (feedstocks) may be moved by auger feeder, with or without the presence of steam, alkali or other catalysts.

Additional (superheated) steam may be introduced at the reactor (e.g., at the bottom of the reactor. This may be referred to as bypass steam.

Reactivity is favored by using pulverized feedstock materials.

3. Introduction of feed as slurry

Coal or other carbonaceous materials mixed with water. Alkali can be introduced into the slurry.

4. Introduction of alkali may be by

a) impregnation on coal or carbonaceous material

b) introductin as dry powder or granules

c) injection as solution or slurry into feed mixture or reacting mixture

d) introduction into coal-water slurry

The carbonate, bicarbonate, hydroxide or oxide form of sodium or other active metals is used. The preference is sodium carbonate. Other alkali or alkaline earth salts can be added. However, they are not as effective as the alkali carbonates (or bicarbonates, oxides, or hydroxides). On the other hand, neither are they deleterious at the reaction conditions used.

Alkali proportions can be up to and equal or exceed the coal or carbonacous material rate. Preferable proportions are approximately 10% by weight.

The alkali can be injected ahead of or

following pretreatment, but preferably the former.

The alkali can alternately be injected into (superheated) steam entering the (bottom) of the reactor. That is, into the bypass steam.

5. Pretreatment

Control of residence time in the pretreatment section is for the following purposes:

Pyrolitic liberation of sulfur compounds.
Capture of liberated sulfur compounds by reaction with the alkali.
Partial (or complete) reaction of steam with the coal or other carbonaceous materials, a heat absorbing endothermic reaction which influences the further conversion(s) in the reactor(s) proper.

Indirect addition of heat to the pretreatment section to control the above.

The use of steam superheat to control the above.

Addition of oxygen ahead of or in the pretreatment section to control the above.

Further addition of steam ahead of or in the

superheat section to control the above.

Addition of carbon dioxide or other oxidizing agents ahead of or in the superheater section to control the above. Addition of hydrogen and/or carbon dioxide ahead of or in the superheater section to control the above.

Temperatures in the superheater section may range from the initial feed temperature, through 700°F, and on up to 1200-1500°F. This will depend upon the degree of reaction occurring, which is also related to the residence time. Residence times will be measured in seconds or minutes, depending upon the degree of reaction to be incurred and the rate of heat addition.

It is preferable in pretreatment, and at all other points in the reacting system, to stay at temperatures below the melting point of the alkali or ash. For sodium carbonate, this is 1564°F.

Fresh or recycled catalyst may be injected ahead of or following pretreatment.

Carbon monoxide, carbon dioxide, or other reactants or products, or other materials, may

be injected ahead of or following pretreatment.

The coal may alternately be fed and moved through the pretreatment section by a screw or auger, and in turn be fed directly into the reactor, preferably at the side near the bottom. The steam in whole or in part may accompany the coal, as may the alkali and other catalysts.

Part of the steam may bypass the pretreatment section, and be superheated in a separate superheater section. This steam may be introduced at the bottom of the reactor (as through a perforated plate). Part or all of this alkali may be introduced with the steam.

The preceding provides an alternate way to feed the reactor and also provides a means to control the $H_2/CO/CO_2$ ratio entering the reactor.

6. Use of multiphase reactor-contactor

The reactor may be in the shape of a vertical cylinder, or in the shape of a vertical cylinder with XXXXX bottom, or in other suitable shapes.

Feedstock plus alkali entrained in steam may be fed at the bottom of the reactor, as previously described. Single or multiple ports can be used.

Or the feedstock, with or without alkali, may be fed into the side or bottom of the reactor (e.g., via a screw or auger), as also previously described. The steam may be fed in whole or in part with the coal, and/or injected as bypass steam at or near the reactor bottom. A distributor plate may be used near the bottom of the reactor, or single or multiple injection ports may be used.

Recycle or other steams may be injected at the reactor, including fresh or recycled catalyst(s).

The multiphase reactor-contactor is characterized as follows:

Solid or liquid reactant phase(s) entrained in gaseous or liquid reactant phase(s). That is, more dense phase(s) entrained or suspended in less dense phase(s).

Ebullating (or fluidized) bed of a catalyst or other solid phase.

Temperature control in reactor

High enough to minimize carbon deposition on catalyst phase

Low enough to minimize formation of CO (and $H_2$)

Range of 1200°F through 1500°F

The reactor vessel may be heated or cooled, but is preferably adiabatic.

Reactor residence times are measured in seconds and up to a minute or more.

The flow space above the expanded catalyst bed is minimized in order to prevent the reforming or further reactions of the effluent gases.

The effluent gases are subjected to a quick quench, direct or indirect, to stabilize the reaction mixture and prevent further reaction. The outlet quench temperature is preferably 900°F or below, though it may be higher if the residence time is short enough during solids separation and before and during the cooling-condensation.

If the residence times are short enough in solids separation and during cooling-condensation of the reactor effluent gases, the separate quench can be eliminated. The

gaseous effluent from the reactor, or from the quench, is subjected to solids separation (via cyclone, filter, and/or baghouse, depending upon temperature). This gaseous effluent from solids separation is sent to a cooler-condenser, followed by a receiver or separator.

The gaseous effluent from solids separation may be utilized as is.

The gaseous effluent from the reactor, or from the quench, may be used as is.

The reactor may serve as a disengagement section, particularly for the case of catalyst recycle.

Internals may be employed in the reactor vessel, including screws or augers, which move the solids while contacting the gaseous (or liquid) phase.

7. Holdup

Extension of pretreatment, residence time by use of holdup vessel: heated, cooled, or adiabatic. Injection of additional alkali: preferably as dry powder or granules, but also as solution or slurry.

Injection of oxygen to control reactions.

Injection or recycle oxidizing agents to

control reaction and sulfiding: steam, carbon dioxide, or other oxidizing agents.

Injection or recycle of reducing agents to control reactions and sulfiding: hydrogen and/or carbon monoxide, or other reducing agents.

Holdup may serve as a disengagement section, particularly for the case of catalyst recycle.

Internals such as screws or augers may be used to move the solids in and/or through holdup.

8. Backup

Backup is used as an extension of holdup. It involves the following aspects:

Contacting effluent stream from pretreatment or holdup with iron oxide pellets or other agents in an ebullating (fluidized) bed for additional sulfur capture.

Contacting solids-free effluent gas stream from pretreatment or holdup with iron oxide pellets or other agents in a fixed bed for additional sulfur capture.

Contacting effluent stream as above for the purposes of adjusting the $H_2/CO/CO_2$ ratios.

Contacting the effluent stream as above for

the purposes of causing methanation or Fischer-Tropsch type reactions to occur.

Regulating temperature externally to control the above reactions.

Regulating temperature internally by the addition of oxygen or air, steam or carbon dioxide, or other oxidizing agents.

Regulating temperature internally by the addition of hydrogen and/or carbon monoxide or other reducing agents.

Internals such as screws or augers may be used to move the solids in and/or through backup.

9. Interstage Gas Treatment

The gaseous effluent from pretreatment, holdup, and/or backup may or may not be subjected to solids separation (via cyclone, filter and/or baghouse).

The gaseous effluent from pretreatment, holdup, and/or backup may or may not be passed through a cooler-condenser, followed by a receiver.

The gaseous effluent from solids separation, above, may or may not be passed through a cooler-condenser, followed by a receiver.

Hot (or cold) gas recycle can be used to pretreatment, to the reactor(s), and to other vessels in the system. Additional heat may be added to (or removed from) the recycle stream.

10. Interstage Solids Transfer

The solid phases may be moved between stages or vessels by entrainment in the gaseous (or liquid) phase. Alternately, the solid phase may be moved by the use of a screw or auger, or similar device.

The above applies also to recycle streams.

11. Methanation or Fischer-Tropsch Step

Injection of $H_2/CO$ mixtures, or $H_2/CO_2$ mixtures, or $H_2/CO/CO_2$ mixtures into an ebullating (fluidized) or fixed bed of methanation or Fischer-Tropsch type catalyst, or other catalysts, to produce methane, higher hydrocarbons, and/or oxygenated compounds, depending upon catalysts and conditions.

Water vapor (steam), already present or added, can be used to direct the course of the reaction.

Carbon dioxide, already present or added, can be used to direct the course of the

reaction. Furthermore, water vapor (steam) and/or carbon dioxide are coproduced, depending upon catalysts and conditions. With excess steam, hydrogen is preferentially produced. Reaction pressure is from near atmospheric to 1000 psi or more. Reaction temperature is from near ambient to 1200-1500°F or more, but is preferentially at 700-800°F. Reaction temperature as well as pressure and feed makeup, and injection of other components, all influence the course of the conversion. The reactions tend to be highly exothermic, requiring the use of interstage heat transfer, intrastage heat transfer, or recycle of cooled products. Reactants and injected components can be introduced cooled, of the order of 200°F more or less. The final effluent may be utilized as is, or passed through a solids separation system and/or cooler-condenser with separator or receiver.

The reactants/or reactant proportions may be controlled to produce either or both water (steam) and carbon dioxide, along

with the hydrocarbon and/or oxygenated products. The type and activities of the catalyst(s), as well as operating conditions, will also influence the outcome of the conversion.

12. Alkalki Recovery and Regeneration

Recovered dry solids, including ash or ash-char and alkali plus sulfided alkali, are contacted with water, preferably hot water, to dissolve excess alkali and sulfided alkali. Other alkali-containing waters may be mixed with this stream. This includes aqueous condensate phase(s) which contain alkali carryover.

The solution so formed can be separated from the undissolved solids, or the mixture can be left as a slurry.

The solution or slurry so formed above is contacted with carbon dioxide. The contacting is preferably done with heated solution or slurry, at or near the boiling point. Steam can be introduced to heat the solution or slurry, preferably along with the carbon dioxide.

The steam so introduced above also provides a stripping action for the sulfur compounds

converted or regenerated by the action of the carbon dioxide.

The action of the carbon dioxide (and steam or water) serves to regenerate the sulfided alkali to the unsulfided form (e.g., sodium carbonate).

The solution or slurry of excess alkali and regenerated alkali is recycled or recirculated to the point(s) of injection or use.

The solution or slurry can be evaporated further to concentrate the alkali for injection.

The solution or slurry, but preferably solution, can be recycled to the steam generator or boiler, to provide a part of the steam requirement. Alternately, a separate steam generator or boiler can be used, designed for the purposes intended.

13. Recovery of Ammonia and Other Water Soluble Gases

The aqueous condensate phase(s) will tend to contain ammonia and other water-soluble gases, and their derivatives. The extent will depend upon the source of the carbonaceous materials, the extent of

pretreatment, etc., and the type and condition of conversion, even catalysts. Even carbon dioxide and hydrogen sulfide will tend to dissolve to a limited extent, and may combine with other components, or exist as ionic mixtures. For instance, ammonia and hydgogen sulfide react to form ammonium sulfide, which is very soluble in water. The aqueous phase is heated, indirectly or directly (with steam) to drive off the ammonia and other gases whose solubility is reduced at higher temperatures. The ammonia is then selectively absorbed for further recovery and processing.

Other gases which may occur, such as hydrogen cyanide, may also be selectively absorbed and treated.

The remaining aqueous phase, which may contain alkali and/or sulfided alkali, is sent to alkali recovery and regeneration.

14. Disposition of Ammonia and Nitrogen

Ammonia and other gases which are carried over in the off-gases leaving the partial condenses and separator will tend to be absorbed (or absorbed) during acid gas recovery -- depending upon the acid gas

process used. While ammonia solutions, for instance, serve or a meduim for acid gas recovery, with other solution or solvents the ammonia will tend to be stripped and appear with the $H_2S$ (and $CO_2$) in the conversion to elemental sulfur. Depending upon the activity of the catalyst, nigrogen may ultimately be produced. ($NH_3$ combusts to nitrogen and water.)

During the conversion of the coal or other carbonaceous material, there is also evidence that molecular nitrogen is produced instead of or in addition to ammonia and other nitrogen compounds. This will depend upon catalyst(s) and conditions.

15. Recovery of Free Sulfur

Any suspended free sulfur which may occur in the condensate as the result of oxidation-reduction reactions is recovered by heating the aqueous suspension above the melting point of sulfur (250°F). The sulfur is drawn off as a separate liquid phase and sent to washing and recovery. Flotation methods using air and other gases are alternately used.

16. Control of Activity of Methanation or Fischer-Tropsch Type Catalysts

This pertains to the control of sulfiding, carbon deposition, and the level of oxidation or reduction of the catalyst components as follows:

Injection or recycle of oxygen or air, steam, carbon dioxide and/or oxidizing agents into reacting mixture.

Injection or recycle of hydrogen and/or carbon monoxide or other reducing agents into reacting mixture.

Circulation of catalyst into a regenerator vessel(s), with recirculation of catalyst to reactor vessel(s).

Injection or recycle or air, steam, carbon dioxide and/or other oxidizing agents into regenerator.

Injection or recycle of hydrogen and/or carbon monoxide or other reducing agents into regenerator.

17. Recovery and Regeneration of Methanation or Fischer-Tropsch Type Catalysts

Breakup and ablation of the methanation or Fischer-Tropsch type catalysts, or other similar catalysts, results in carryover.

This carryover collects with the ash or ash-char (and alkali) in the dry solids separation system (cyclone, filter, and/or baghouse). It also collects as part of the ash or ash-char carryover in the separator or receiver following the cooler-condenser(s).

The dry solids and/or sludge are contacted with acidic or basic solutions to dissolve the soluble catalyst components.

The resulting solution is contacted with oxidizing agents such as oxygen or air, steam or carbon dioxide to remove sulfur as the oxide(s).

The catalyst component, precipitated at a higher level of oxidation, is washed and partially reduced with hydrogen and/or carbon monoxide or other reducing agents to restore activity.

Alternately, the resulting solution is contacted with reducing agents such as hydrogen and/or carbon monoxide to remove sulfur as hydrogen sulfide, principally. The catalyst component, precipitated at a lower level of oxidation, is washed and partially oxidized with air or oxygen, steam, carbon

dioxide, and/or other oxidizing agents to restore activity.

The activated catalyst is recycled to the reactor(s) or regenerator(s).

Inasmuch as activation will also occur in the reactor(s) and regenerator(s), the inactive catalyst may also be recycled.

At startup, catalysts at a higher level of oxidation may be activated by partial reduction, as above.

At startup, catalysts at a lower level of oxidation may be activated by partial oxidation, as above.

Also, during startup, it is not necessary to activate in place, since the catalyst can become activated from contact with gaseous intermediate products, or from the injection of reducing agents or oxidizing agents, as described above.

18. Separation of Methanation or Fischer-Tropsch-Type Catalysts

Catalyst components may be separated from ash components and other components by differences in magnetic susceptibility: e.g., magnetic, paramagnetic, and nonmagnetic. In fact, ash components per se can be so

separated. This involves the following: Heating solids carryover, if necessary, to increase component magnetic susceptibility. Temperatures up to and beyond roasting temperature, circa 3000°F, are involved. Use of char in the solids carryover to supply the heat by combustion, or partial combustion, as per the above. Adjustment of magnetic field to distinguish between components of differing magnetic susceptibilities.

Repetition of the above for successive separation. Other separation procedures used in mining and extractive metallurgy can also be employed: classification due to differences in density and particle size; flotation; etc.

19. Sulfur Recovery

The bulk of the sulfur compounds liberated, mainly as hydrogen sulfide, will be captured by the alkali, and will again appear during the regeneration of the alkali (solutions or slurries) with carbon dioxide (and steam). The mixture of hydrogen sulfide and other compounds so produced, plus the excess carbon dioxide (and steam)

used, is sent to a Claus unit or similar unit for conversion to elemental sulfur. For lower concentrations, a Stretford unit or similar unit may be used to separate out the hydrogen sulfide and other sulfur compounds, and further convert them to elemental sulfur.

Also, during control of sulfiding by the addition of oxygen or air, steam, carbon dioxide, and/or other oxidizing agents, elemental sulfur may appear at reaction conditions.

The elemental sulfur so produced, above, is collected as the liquid or solid phase at the separator or receiver following the cooler-condenser(s), at the appropriate points.

Steam (or waste heat) can be produced at the Claus unit (or its equivalent) -- a result of the oxidation of hydrogen sulfied ot yield sulfur.

20. Oily Phase

Depending upon the operations of pretreatment and subsequent reactions, and the type of catalyst and reaction conditions, and residence times, etc., an oily phase or

phases may make an appearance in the separator or receiver following the cooler-condenser(s). This oily phase or phases may consist of pyrolysis products which have not been further converted, and/or may consist of higher hydrocarbons and/or oxygenated compounds resulting from Fischer-Tropsch type reactions, or other reactions. Sulfur and nitrogen compounds, for instance, may be part of or dissolved in the oily phase(s).

The oily phase or phases produced, if any, are recycled to appropriate points in the reaction sequence, in part or in total: to the feed mixture, to pretreatment, to the multiphase reactor-contractor, to holdup, to backup, and/or to points between.

The part not recycled becomes a net product, for fuel or for further refining.

21. Condensate Temperature

The condensate temperature is regulated to control the disposition of hydrogen sulfide and other gases.

At higher temperatures, near the boiling point of the aqueous condensate (circa 212°F), even in the presence of alkali

carryover, hydrogen sulfide will tend to remain in the gas phase in the presence of carbon dioxide. Other water soluble gases such as ammonia, however, will also tend to be stripped from solution. At lower temperatures, circa 100°F for example, the hydrogen sulfide will tend to stay in solution in the presence of alkali, notably as the alkali sulfide. Other water-soluble gases such as ammonia will also tend to remain dissolved in the aqueous condensate.

22. Nitrogen Control

The addition of oxidizing agents or reducing agents, and the presence of oxidizing or reducing reactions will not only affect the form of sulfur produced, but will also affect the form of nitrogen. Therefore, by controlling the level of oxidation-reduction occurring during pretreatment and conversion, and during holdings and backup -- which also affect sulfur control -- the form of nitrogen produced can be in part controlled -- including the production of molecular nitogen vs. the production of ammonia, etc. The above also applies to other components

which may be present, such as chlorine and fluorine and their compounds.

23. Char Recycle

Untreated char solids can be recycled to the reactor for further conversion. That is, part of the solids residue from solids separation can be recycled either or both to the pretreatment section or to the reactor. This may include catalyst carryover: Classification methods can be used to effect a char/ash/catalyst separation. Solids residue from the cooler-condenser-separator can also be recycled.

As also mentioned elsewhere, the oily phase(s) may be recycled for further conversion.

24. Lean Oil Absorption

Temperature, pressure, catalyst, residence time and other factors affect the product makeup leaving the conversion step.

While a water-wash, or aqueous solutions of active components may be used to remove certain types of compounds, other types of compounds may be removed and collected by using a lean oil absorption step. This is appropriate for hydrocarbons or

hydrocarbon-soluble compounds. This step may proceed or follow the gas/liquid separator.

25. Acid Gas Recovery and Separation

The acid gases carbon dioxide and hydrogen sulfide produced at different stages during conversion and/or processing may be recovered and separated by known techniques. The acid gases are removed simultaneously and/or selectively (usually the selectivity is toward hydrogen sulfide) and are regenerated, usually by heating, stripping with live steam, and/or flashing to a lower pressure. Solutions (usually aqueous) or solvents are the media employed. These methods include the use of sodium carbonate solutions (vacuum carbonate process) and hot potassium carbonate solutions (hot carbonate or Benfield process). Of particular note is the use of monoethanolamine or diethanolamine solutions. The former, however, reacts irreversibly with carbonyl sulfide which may be present. Triethanolamine solutions may be used, and may also be employed to introduce a degree of selectivity between hydrogen sulfide and

carbon dioxide.

The Selexol process (using the dimethyl ether of propylene glycol as solvent) successfully removes both carbon dioxide and hydrogen sulfide. At different operating conditions, hydrogen sulfide may be removed selectively, occasioning the use of a dual cycle for selective removal. That is, two stages are employed.

Ammonia solutions can be used, and under appropriate conditions will show selectivity in favor of hydrogen sulfide.

Alkacid (alkazid) "M" solutions will remove both hydrogen sulfide and carbon dioxide. The "dik" form removes hydrogen sulfide selectively.

Iron oxide pellets will remove hydrogen sulfide selectively, to form the sulfide, and can be regenerated.

Physical adsorbents can be used for both hydrogen sulfide and carbon dioxide, and under appropriate conditions will provide selectivity. This includes molecular sieves. Of particular note is "steady-state adsorption", embodied by the

Hypersorption process for hydrocarbon recovery, in which a downward moving bed of solid adsorbent particles is contacted with the gases to be separated. A regeneration or recovery step follows. Other embodiments are also conceivable aside from fixed beds: e.g., fluid beds and entrained system.

For more concentrated streams of hydrogen sulfide, the Claus process or its equivalent can be used to produce elemental sulfur -- which in a manner of speaking is a selective hydrogen sulfide recovery method.

For more dilute concentrations of hydrogen sulfide, the Stretford process or its equivalent may be used to selectively remove and convert hydrogen sulfide to elemental sulfur.

26. $CO_2$ - Injection

The byproduct or coproduct carbon dioxide can be injected into oil-bearing formations to increase the recovery. The $CO_2$ dissolves (become miscible with the oil) to lower the viscosity and increase mobility. At the same time, or otherwise,

the carbon dioxide can act to pressure the formation and produce an immiscible or gas drive. It should be kept in mind, that as $CO_2$ is progressively dissolved in oil, a point is reached where a second phase is formed. At lower pressures, this second phase is a $CO_2$ - rich gas phase. At higher pressure, it becomes a $CO_2$ rich liquid phase.

The phases formed, and the proportions, will all affect the degree of additional oil recovery.

There is also the possibility that still other phases may make an appearance. For example, asphalt-like materials may form as a distinct phase, being less soluble in $CO_2$ - rich mixtures.

The successful application of $CO_2$ - injection therefore also depends upon the nature of the oil as well as carbon dioxide proportions and pressures (and temperatures).

27. Action of Hydrogen Sulfide in Enhanced Recovery.

It is not necessary to remove the hydrogen sulfide from the carbon dioxide for the purpose of enhanced oil recovery. For hydrogen sulfide itself acts in similar

fashion to carbon dioxide, also enhancing oil recovery.

28. $CO_2$/Water Injection

In the event the byproduct or coproduct carbon dioxide is used for enhanced oil recovery, the waste-water (condensate) phase can be injected into the oil-bearing formation simultaneously or alternately with the carbon dioxide. This combination of water drive (flooding) and $CO_2$-injection sometimes increases the recovery over $CO_2$ -injection above (or water-injection alone).

As a further note, the alkali content of the waste water will further assist displacement of the oil (caustic flooding).

29. Hot Gas Injection

The entire reactor overhead can be injected into the oil-bearing formation during enhanced oil recovery operations. The effectiveness will depend upon the relative pressure levels and the temperatures, and will be limited by equipment considerations.

Alternately, the $CO_2$ coproduct may be reheated via waste-heat exchange (e.g., the

reactor overhead cooler-condenser system), to achieve a higher injection temperature.

30. Injection of Combustion Products

Under certain circumstances, in enhanced oil recovery, the combustion products from the coal-fired boiler, or from other fuel-fired auxiliaries, may be pressured and injected into the oil-bearing formation. This may be in lieu of, or in support of, carbon dioxide injection. Combustion gases, which are composed principally of nitrogen with lesser amounts of carbon dioxide, may act as the gas drive during immiscible displacement. At higher pressures, the gases tend to dissolve in the oil, to produce a miscible drive. The combustion products may be injected simultaneously or alternatively with $CO_2$-injection, or injected apart. A complication is that oxygen left in the combustion products may promote the growth of bacteria in the formation, and which could block the mobility of the oil (plug the pores of the formation). This is also a potential problem with using nitrogen obtained from the cryogenic

(low-temperature) separation of air.

31. Oxygenated Compounds in Enhanced Oil Recovery

Oxygenated compounds such as alcohols may act as surfactants, in this way or otherwise increasing the mobility of the oil and its recovery.

Thus the use of iron or similar catalysts can be used also to produce alcohols and other oxygenated compounds, along with or in lieu of hydrocarbons.

These alcohols or oxygenated compounds so-produce can therefore be injected into the oil-bearing formation to increase recovery.

These materials may be injected with the aqueous phase (condensate produced) and in fact are soluble, partially soluble, or form emulsions with water.

32. Higher Hydrocarbons in Enhanced Oil Recovery

Higher hydrocarbons produced, particularly propane or LPG fractions, can be used in enhanced oil recovery. These hydrocarbons tend to mix (become miscible) with the heavier oil and increase the recovery of the oil.

33. Hydrogenation Step

The separation of carbon dioxide from

$H_2/CO_2$ mixture will yield essentially pure hydrogen for other purposes. These include hydrogen for fuel and for hydrogenation reactions.

Notable among the latter in the production of ammonia by reactions with nitrogen cryogenically separated from air, or recovered via the $CO_2$ scrubbing of combustion gases.

34. Waste-Disposal

Ash-char solid residues may be sent to holding pits or other solid-waste disposal systems. These solids may also contain catalyst carryover, and may be processed for catalyst recovery as previously indicated.

Bulk quantities of spend catalyst may be sent to refining facilities.

Waste-water, e.g. the aqueous phase from the partial condenation of the reactor overhead, may be sent to (lined) holding ponds. Alternately, the aqueous phase may be recycled to produce (reactor) steam, as previously indicated. The use of holding ponds is particularly apt for smaller installations.

Evaporation of the water may be accelerated by using cooling towers or spray ponds. The solids present become a sludge at the bottom of the pond.

As an option, the holding pond(s) and pit(s) may be combined. The aqueous phase may be treated by introducing chemical and/or biological additives, and/or by aeration.

35. Combustion of Char

Any unreacted char residue may be combusted in support of the process or utilized otherwise.

The char may be washed to remove alkali or other soluble materials, as in the case of alkali recovery.

The char may be combusted in a cyclone burner or furnace. This is particularly appropriate if alkali components are present, naturally or added. Cyclone burners are designed to operate at slagging conditions, which is enhanced by the presence of alkalies.

The char may also be mixed with fresh coal, which supplies additional volatiles to support combustion. The char may also be

0112117

mixed with gaseous or liquid fuels.

36. Separation of Ash/Char

The washing or extraction of the ash/char mixture to remove water-soluble alkali and other water-soluble components can also serve to separate the ash/char components. That is, water elutriation will distinguish and separate the particle by density and size.

Flotation and other mineral separation processes can also be used.

The above operations are enhanced by increasingly small particle sizes, which tend to fragment and distinguish between the dissimilar char and ash components.

37. Waste-Heat Utilization

All streams which are cooled, or where condensation occurs, may appropriately be used to heat other streams, directly or indirectly.

All such streams, above, may be used to generate steam where the temperature levels are appropriate.

Any streams produced or discharged, which are at sufficient temperature levels, maybe utilized for the above purposes.

38. Slurry Pipelining

The slurry feed system will accommodate coal/water mixtures such as transported by slurry pipeline. The coal/water proportions are the same.

Moreover, alkali water, or even brackish or salt water can be accommodated. The presence of alkali, of course, contributes to calaytic activity. However, the presence of salts such as sodium chloride do not adversely affect the conversion. Such salts may even have a slight catalytic activity. At the low or moderate temperatures of processing the salts will remain inert and not melt, dissociate or decompose -- matters which become of consequence in high-temperature processing such as in combustion.

39. Package Units

Smaller plant capacities (for instance, 100-200 tons per day of coal or other carbonacious materials, more or less) can be accommodated by skid-mounted package units. These units are preferably shop fabricated and pre-assembled for the most part. These

package units constitute a unique embodiment of the invention.

CLAIMS:

1. A method for the production of gaseous and liquid hydrocarbons and carbon dioxide from carbonaceous materials comprising the steps of:

mixing pulverized carbonaceous materials, steam and alkali catalyst and raising the temperature to an operating temperature range of 1200-1500°F and where said alkali catalyst has essentially a higher fluxing point than the operating temperature of the mixed materials;

allowing sufficient residence time in said mixing step for the reaction of the components to release hydrogen sulfide and produce carbon monoxide, carbon dioxide and hydrogen and for providing alkali capture in sulfide form;

injecting the output of said mixing step into a fluidized or ebullating bed of catalyst comprised of Group VIII transition metal compounds at a temperature of 1200-1500°F and a pressure in a range from atmospheric to 1000 psig. for producing carbon dioxide and hydrocarbons as well as residue solids; and

treating the off-gases and solids carryover from said fluidized or ebullating bed in a quench/cooler to stop the reactions and provide

gaseous and liquid hydrocarbon and carbon dioxide products.

2. The method according to Claim 1 wherein the ebullating bed comprises a catalyst of tungsten or molybedenum compounds.

3. The method according to Claims 1 or 2 where the operating temperature of the mixing step is in the range of 1300°F to 1400°F.

4. The method according to Claims 1 or 2 wherein the step of treating the off-gases and solids in a quench/cooler is followed by the step of separating out the solids from gases.

5. The method according to Claim 1 wherein an oxidizing agent is injected into the mixing step to remove sulfur by oxidation.

6. The method according to Claim 1 wherein a reducing agent is injected into the mixing step to remove sulfur by reduction.

7. The method according to Claim 1 wherein the alkali catalyst is regenerated by dissolution in water followed by injection of steam and carbon dioxide.

8. The method according to Claim 4 wherein the off-gases are separated for the recovery of carbon dioxide.

9. The method according to Claim 5 wherein the oily phases of the off-gases are recycled for removal of ammonia and free sulfur.

10. The method according to Claim 1 wherein the carbonaceous material is a solid in the form of coal, coke, char, lignite, peat, gilsonite, leonardite, oil shale, tar sand and oil sand.

11. The method according to Claim 1 wherein the carbonaceous material is a solid in the form of wood and wood products, byproducts, and wastes, and in the form of plant materials including agricultural crops and crop products and residues, in the form of organic municipal wastes and sewage sludge, and in the form of animal manures.

12. The method according to Claim 1 wherein the carbonaceous material is a liquid in the form of oils and tars.

13. The method according to Claim 1 in which the carbonaceous material and alkali or alkaline materials are premixed and injected into the steam.

14. The method according to Claim 1 wherein the carbonaceous material is mixed with water and alkali to form a slurry and passed through a vaporizer to produce steam, which entrains the coal and alkali.

15. The method according to Claim 1 wherein the ebullating or fluidized catalyst in the reactor is recycled to a separate regenerator vessel for regeneration or activation.

16. The method according to Claim 1 wherein two or more reactor vessels are employed, which are cycled between functioning as reactors and as catalyst regenerators.

17. The method according to Claim 1 wherein oxygen, steam and carbon dioxide are added to the mixing step.

18. The method according to Claim 1 wherein hydrogen and carbon monoxide are added to the mixing step.

19. The method according to Claim 1 wherein the residence time is between 10 seconds and one hour.

20. A method for the production of gaseous and liquid hydrocarbons and carbon dioxide from carbonaceous materials comprising the steps of:

mixing pulverized carbonaceous materials, steam and alkali catalyst and raising the temperature to an operating temperature range of 1200-1500°F and where said alkali catalyst has essentially a higher fluxing point than the operating temperature of the mixed materials;

allowing sufficient residence time in said mixing step for the reaction of the components to release hydrogen sulfide and produce carbon monoxide, carbon dioxide and hydrogen and for providing alkali capture in sulfide form;

injecting the output of said mixing step into a holdup vessel for additional residence time and adding additional alkali catalyst to such holdup vessel;

separating out solids and gases from the output solids and gases from the output of said holdup vessel and subjecting the output gases to an iron oxide catalyst in a backup vessel to regulate the $H_2/CO/CO_2$ ratios and to control sulfur content;

separating out the solids and gases from the backup vessel and subjecting the output gases to a Group VIII catalyst.

21. The method according to Claim 1 wherein the quenching temperature is 900-1000°F.

22. The method according to Claim 1 wherein oxygen, steam and carbon are added to the ebullating bed.

23. The method according to Claim 1 wherein hydrogen and carbon monoxide are added to the ebullating bed.

24. The method according to Claim 1 wherein the Group VIII catalyst is regenerated by use of oxygen or steam.

25. The method according to Claim 1 wherein the Group VIII catalyst is regenerated by the use of a hydrogen or carbon monoxide.

26. The method according to Claim 4 wherein the solids are subjected to magnetic separation to collect magnetic metal materials.

27. The method according to Claim 4 wherein the solids are subjected to floatation to collect metal materials.

28. A method according to Claim 1 or 20 for producing hydrogen and carbon dioxide only by adding steam in excess of the steam/coal ratio of 1.5 to 1 up to a ratio of 2.5 to 1.

29. A method according to Claim 1 or 20 for producing oxygenated compounds by the additional use of zinc and copper catalysts.

30. A method according to Claim 28 and further including the step of using an alumma/silica catalyst.

31. A method according to Claim 29 wherein the output gases are subjected to a catalyst of tungsten or molybdenum compounds.

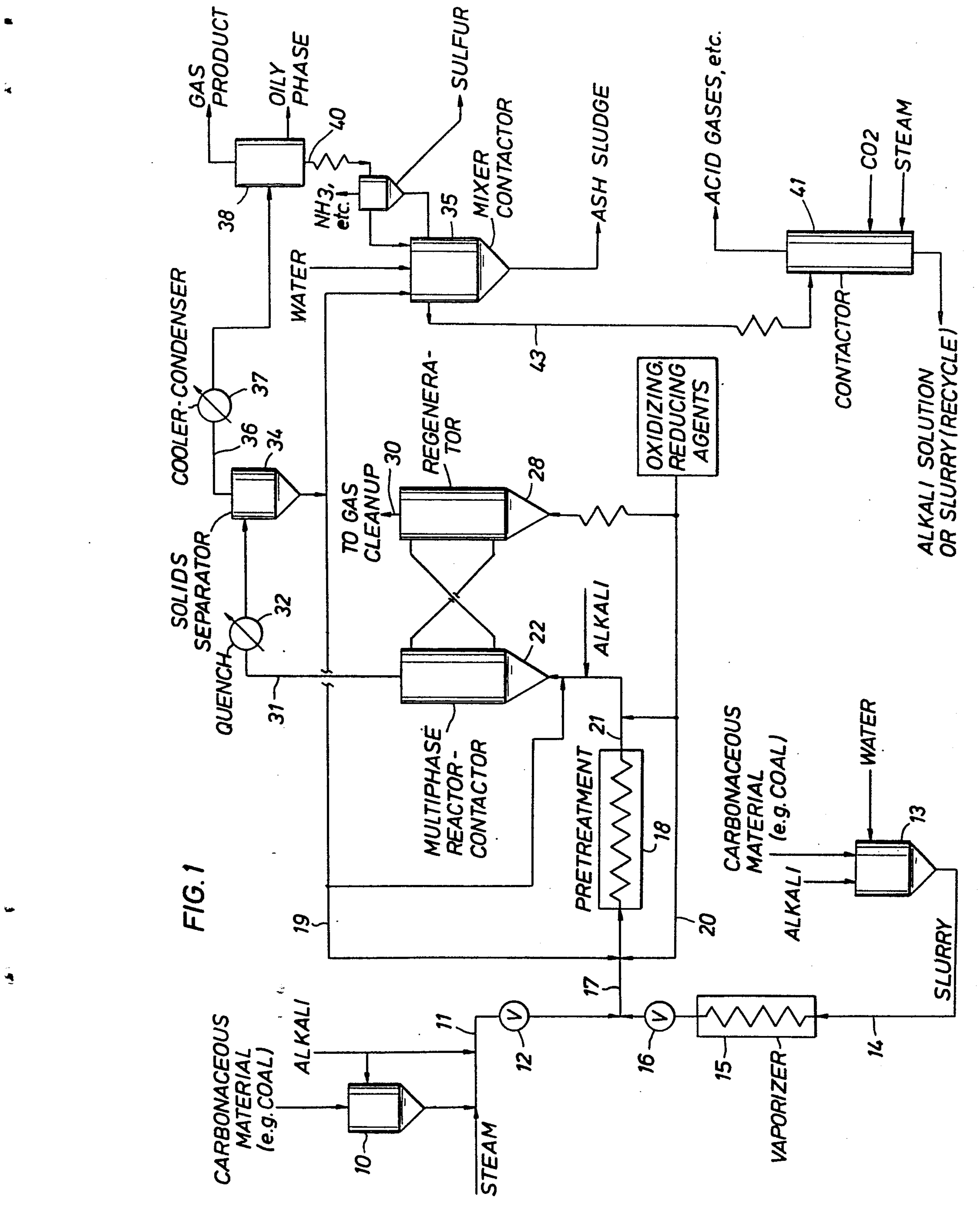
FIG.1
CARBONACEOUS MATERIAL (e.g.COAL)
ALKALI
STEAM
10
11
12
V
17
16
15
VAPORIZER
14
SLURRY
13
WATER
CARBONACEOUS MATERIAL (e.g.COAL)
ALKALI
19
20
PRETREATMENT
18
21
MULTIPHASE REACTOR-CONTACTOR
22
ALKALI
QUENCH
31
32
SOLIDS SEPARATOR
34
36
COOLER-CONDENSER
37
TO GAS CLEANUP
30
REGENERATOR
28
OXIDIZING, REDUCING AGENTS
38
GAS PRODUCT
OILY PHASE
40
SULFUR
NH3, etc.
35
MIXER CONTACTOR
WATER
ASH SLUDGE
43
ACID GASES, etc.
41
CO2
STEAM
CONTACTOR
ALKALI SOLUTION OR SLURRY (RECYCLE)

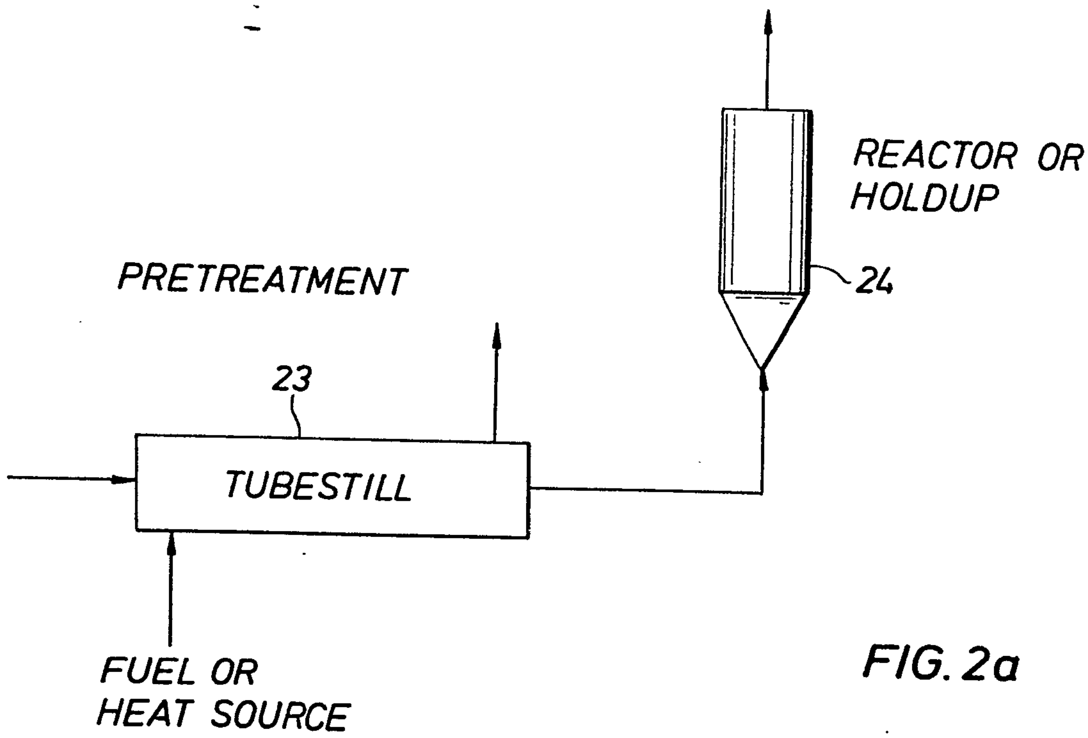

REACTOR OR
HOLDUP
24
PRETREATMENT
23
TUBESTILL
FUEL OR
HEAT SOURCE


FIG. 2a

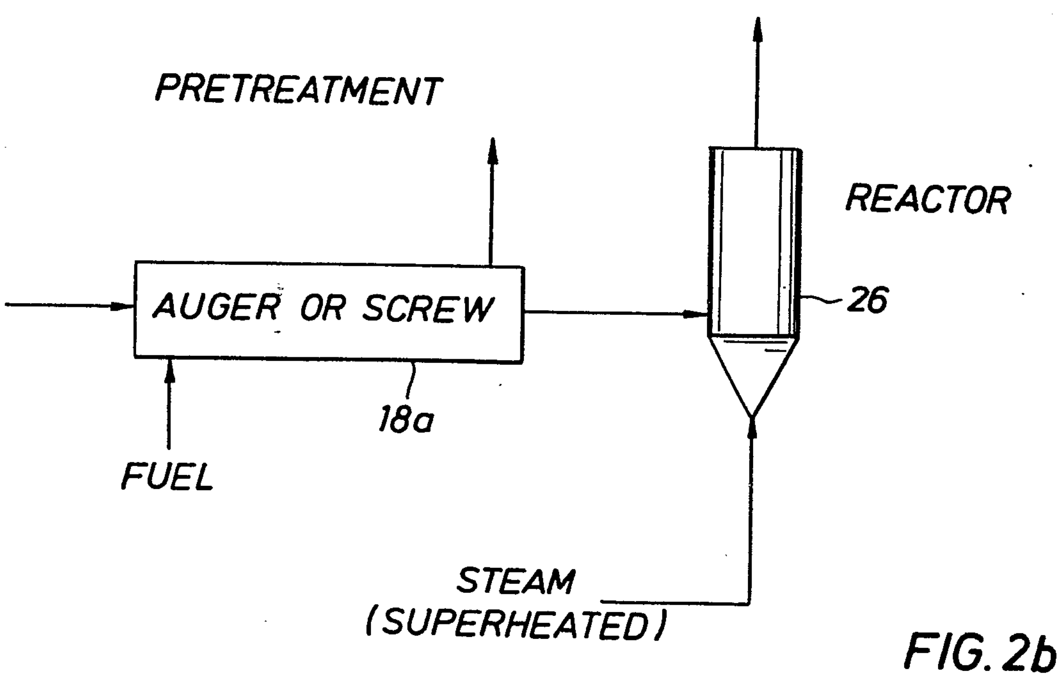

PRETREATMENT
REACTOR
AUGER OR SCREW
26
18a
FUEL
STEAM
(SUPERHEATED)


FIG. 2b

FIG. 3

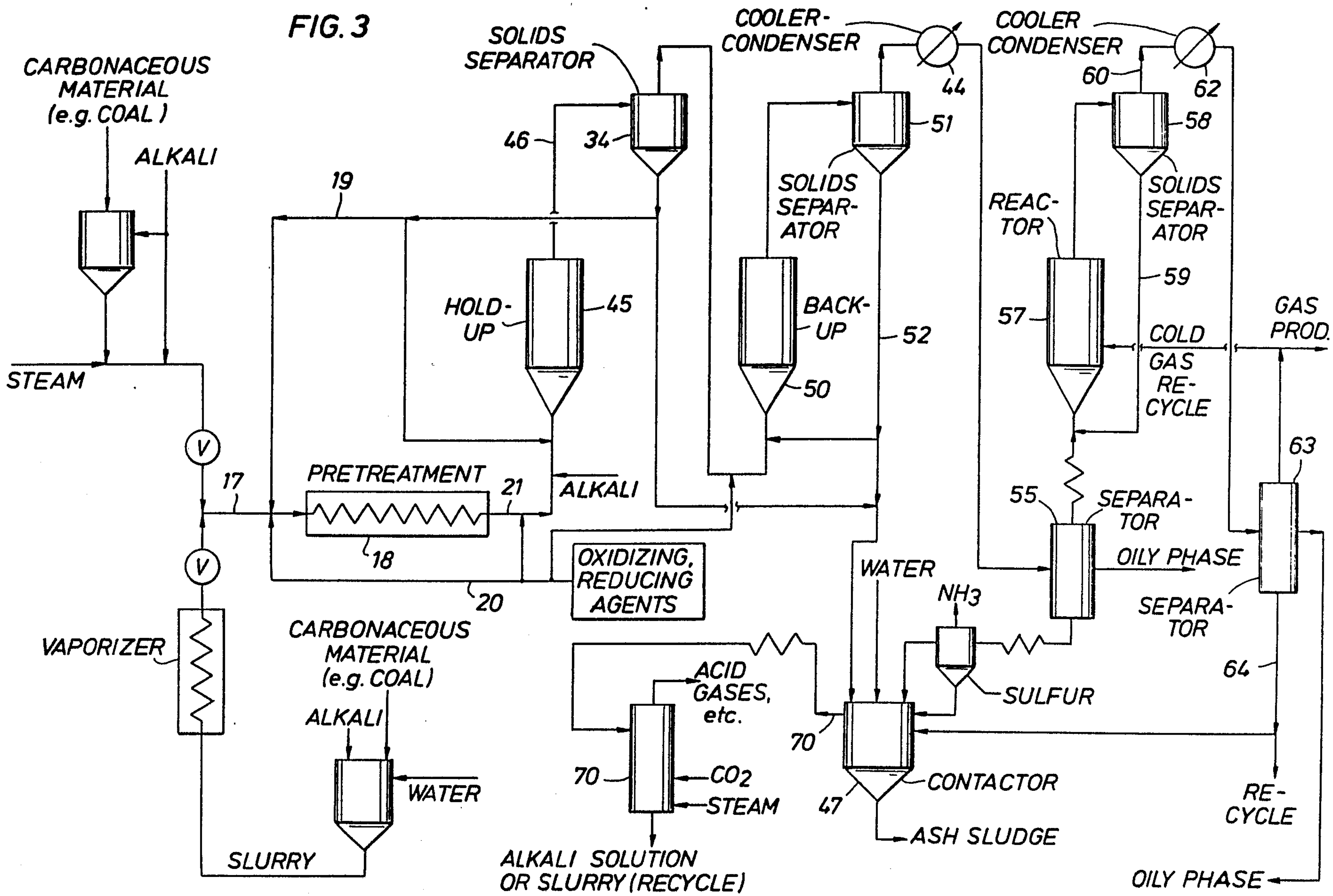
CARBONACEOUS MATERIAL (e.g. COAL)
ALKALI
STEAM
V
17
V
VAPORIZER
SLURRY
CARBONACEOUS MATERIAL (e.g. COAL)
ALKALI
WATER
19
PRETREATMENT
18
21
ALKALI
20
OXIDIZING, REDUCING AGENTS
SOLIDS SEPARATOR
46
34
HOLD-UP
45
COOLER-CONDENSER
44
51
SOLIDS SEPAR-ATOR
BACK-UP
50
52
COOLER CONDENSER
60
62
58
SOLIDS SEPAR-ATOR
REAC-TOR
57
59
COLD GAS RE-CYCLE
GAS PROD.
63
55
SEPARA-TOR
OILY PHASE
SEPARA-TOR
64
RE-CYCLE
OILY PHASE
WATER
NH3
SULFUR
70
CONTACTOR
47
ASH SLUDGE
ACID GASES, etc.
70
CO2
STEAM
ALKALI SOLUTION OR SLURRY (RECYCLE)